(19) Europäisches Patentamt / European Patent Office / Office européen des brevets

(11) **EP 2 783 715 B1**

(12) **EUROPÄISCHE PATENTSCHRIFT**

(45) Veröffentlichungstag und Bekanntmachung des Hinweises auf die Patenterteilung:
**10.05.2017 Patentblatt 2017/19**

(51) Int Cl.:
*A61M 1/36* (2006.01)   *A61M 1/16* (2006.01)

(21) Anmeldenummer: **14159199.0**

(22) Anmeldetag: **12.03.2014**

(54) **Verfahren zur Erfassung einer Rezirkulation in einem arteriovenösen Shunt während laufender Hämodialyse und Dialysesystem**

Method for detecting recirculation in an arteriovenous shunt during ongoing haemodialysis and dialysis system

Procédé de détection d'une recirculation dans un shunt artérioveineux pendant une hémodialyse et système de dialyse

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.03.2013 DE 102013103221**

(43) Veröffentlichungstag der Anmeldung:
**01.10.2014 Patentblatt 2014/40**

(73) Patentinhaber: **B. Braun Avitum AG**
**34212 Melsungen (DE)**

(72) Erfinder:
• **Krause, Silvie**
  **34212 Melsungen (DE)**
• **Strohhöfer, Christof**
  **34123 Kassel (DE)**

• **Ahrens, Jörn**
  **34225 Baunatal (DE)**
• **Castellarnau, Alex**
  **215021 Suzhou (CN)**
• **Weyer, Sören**
  **52064 Aachen (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
WO-A1-00/24440   WO-A1-2011/026647
DE-A1-102007 056 475   DE-C1- 19 702 441
DE-T2- 69 916 053   US-A1- 2009 054 822

**Beschreibung**

Die vorliegende Erfindung betrifft ein Verfahren zur Erfassung einer Rezirkulation in einem arteriovenösen Shunt eines Patienten während einer laufenden Hämodialyse gemäß dem Oberbegriff des Anspruchs 1 sowie ein Dialysesystem gemäß dem Oberbegriff des Anspruchs 9.

Patienten mit schwerer Niereninsuffizienz oder Nierenversagen sind bekanntlich dialysepflichtig. Hierzu stehen den betroffenen Patienten zwei Dialysemöglichkeiten zur Verfügung, einerseits in einem Dialysezentrum einer Klinik und andererseits - nach Einführung durch den behandelnden Nephrologen und geschultes Fachpersonal - die Selbstdialyse im Heimbereich mit ambulanten Dialyseeinheiten.

Unter dem Begriff "Dialyse" bzw. "Hämodialyse" wird für die Zwecke der vorliegenden Erfindung jegliche chronisch anzuwendende Blutreinigungstherapie verstanden. Diese umfasst Hämodialyse, Hämofiltration sowie Hämodiafiltration.

Zur Durchführung einer Dialyse bei einem Patienten mit chronischer Niereninsuffizienz wird typischerweise operativ ein sogenannter arteriovenöser Shunt gelegt, der eine direkte Verbindung einer Arterie mit einer Vene darstellt. Einer der häufigsten Shunts, die zur Dialysebehandlung gelegt werden, ist der sogenannte Cimino-Shunt, bei welchem - nach Gefäßpräparation - die Arteria radialis mit der Vena cephalica verbunden wird.

Ein derartiger Shunt lässt eine einfache Gefäßpunktion zur Aufnahme der für eine Dialyse erforderlichen Kanülen zu und weist außerdem aufgrund der im Shunt herrschenden Druck und Flow-Verhältnisse einen ausreichenden Blutfluss zur Durchführung einer Hämodialyse auf.

Allerdings wird der Gefäßzugang in Nephrologenkreisen als Achillesferse der Hämodialyse angesehen [San Miguel, S & Chow, J: Vascular dialysis access flow measurement: Early intervention through early detection. J Renal Care:185-191, 2009].

In diesem Zusammenhang berichten australische Nephrologen [San Miguel, S & Chow 2009] über einen Untersuchungszeitraum von November 2006 bis Mai 2007, in dem bei 35 Hämodialysepatienten die Dialysezugänge sonographisch untersucht wurden. Von dem untersuchten Patientenkollektiv lag bei 50% eine signifikante Shunt-Stenose vor, 11% zeigten eine Thrombose, 6% wiesen Aneurismen auf, weitere 6% hatten nicht näher bezeichnete Probleme mit ihrem Zugang und lediglich 19% waren ohne Befund.

Während der Dialyse kann es somit bedingt durch die oben genannten Shunt-Komplikationen zu einem zu geringen Shuntfluss und hierdurch zu einer sogenannten "Rezirkulation" im Shunt kommen. Eine Rezirkulation verursacht den direkten Rückfluss von bereits gereinigtem Blut aus dem venösen Zugang direkt wieder in den arteriellen Zugang zum Dialysator. Dies verursacht eine deutliche Reduzierung der Dialyseeffektivität und kann je nach Ausmaß der Rezirkulation ferner als frühzeitiger Indikator für eine möglicherweise erforderliche Shunt-Intervention oder -Rekonstruktion dienen.

Weil eine Rezirkulation zu einer Verschlechterung der Dialyseeffektivität führt, und damit der Patient sein Behandlungsziel nicht erreicht, wird sich sein Allgemeinzustand beim Vorhandensein einer unbemerkten Rezirkulation auf Dauer verschlechtern. Eine online-Messung, die automatisch durch die Maschine durchgeführt werden kann, würde dem Arzt bei Verdacht auf eine vorliegende Shuntrezirkulation eine Möglichkeit einräumen, diese sofort zu verifizieren oder zu falsifizieren.

Gleichfalls kann eine automatisch durchgeführte Messung, z. B. vor jeder Dialysetherapie, den Arzt oder das Pflegepersonal auf Vorliegen einer Shuntrezirkulation hinweisen. Eine Rezirkulation kann die Folge zweier unterschiedlicher Ursachen sein: Ein Fehler beim Anlegen des Patienten an die Dialysemaschine oder wie erläutert eine Degeneration des Shunts. Der erste Typ der Rezirkulation kann nach Hinweis durch das Dialysesystem meist leicht durch das medizinische Personal korrigiert werden. Beim zweiten Typ kann die Messung der Rezirkulation durch die Dialysevorrichtung dem Arzt wertvolle Hinweise zur weiteren Vorgehensweise geben.

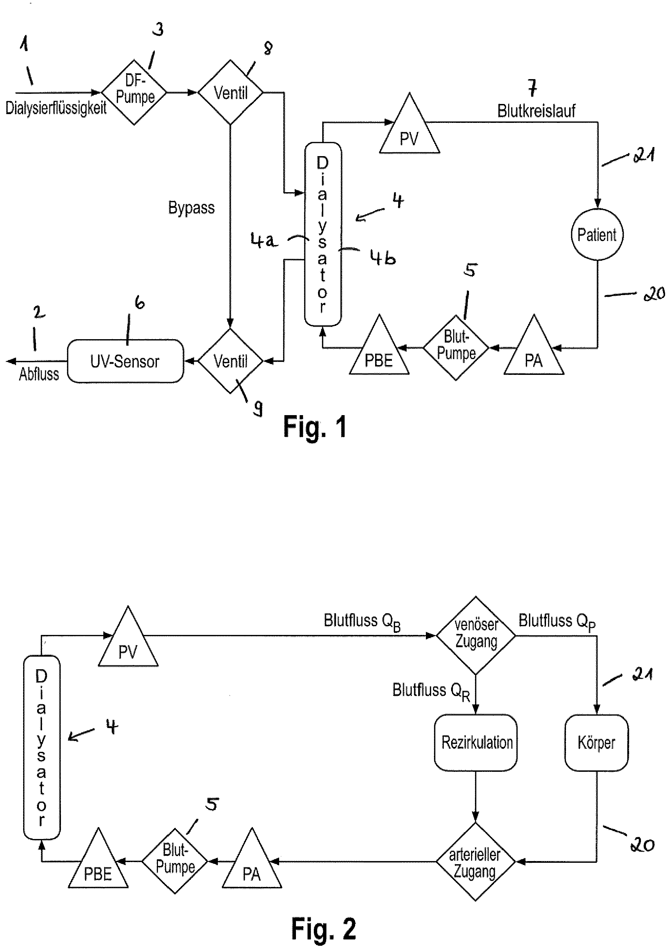

Die Rezirkulation R ist definiert als das Verhältnis der Flüsse von rezirkuliertem Blut Qr und Blutpumpenrate Qb (vgl. Fig. 2) und wird in % angegeben:

$$R = \frac{Qr}{Qb}$$

Zur Messung der Rezirkulation gibt es im Stand der Technik eine Reihe von Ansätzen:

So beschreibt KRIVITSKI 1995 die Theorie und Validierung von Flowmessungen im Dialysezugang mittels eine Verdünnungsmethode durch Bolusinjektion von physiologischer Kochsalzlösung in den Blutstrom des Zugangs eines Patienten und Messung der Blutproteinverdünnung durch Dopplersonographie [Krivitski, N: Theory and validation of access flow measurements by dilution technique during hemodialysis. Kidney International 48:244-250, 1995]. Das Verfahren von KRIVITSKI 1995 basiert auf der Erfassung unterschiedlicher Schallgeschwindigkeiten von Blut und der physiologischen NaCl-Lösung.

Ferner beschreibt Dokument EP 0 886 529 B1 (Gambro Hospital) eine Vorrichtung zur Bestimmung der Blutrezirkulation in einem vaskulären Zugang eines Dialysesystems durch Messung der Hämoglobinkonzentration im extrakorporalen Blutkreislauf der Dialysevorrichtung.

US 7,815,852 B2 (Gambro Lundia) offenbart die Messung eines kardiovaskulären Parameters durch Bolusgabe auf der Blutseite und Bolusdetektion auf der Dialysatseite im Dialysatablauf.

Darüber hinaus beschreibt DE 197 02 441 C1 (Fresenius Medical Care) ein Verfahren zur Bestimmung der Rezirkulation während einer extrakorporalen Blutbehandlung unter Verwendung eines Konzentratbolus' auf der Dialysatseite.

Ferner offenbart DE 195 28 907 C1 (Fresenius AG) ein Messverfahren zur Bestimmung der Rezirkulation unter Ausnutzung von Systemänderungen, die durch kurzzeitiges Umkehren des Blutflusses in der Dialysevorrichtung hervorgerufen werden.

DE 10 2007 056 475 A1 (Fresenius Medical Care) beschreibt ein Verfahren und zugehörige Vorrichtung zum Bestimmen der Rezirkulation in einer Fistel wobei die Rezirkulation durch Veränderung einer chemisch-physikalischen Messgröße bei zwei unterschiedlichen Blutflüssen durch Messung auf der Blutseite der Dialysevorrichtung. Als bevorzugtes Verfahren offenbart DE 10 2007 056 475 A1 ein Thermodilutionsverfahren. Die Erkennung einer Rezirkulation erfolgt bei diesen Dialysegeräten durch eine Temperaturkontrolle (Bluttemperaturmonitor) des Blutes und durch die Bolusgabe eines Temperaturpulses.

WO 00/24440 A1 (Gambro AB) offenbart ein Verfahren und eine Einrichtung zur Messung eines Fluidflusses an einem Zugang. Entnommenes Fluid wird unter Vertauschung von Entnahme- und Rückführposition und jeweiliger Konzentrationsmessung einer Substanz rezirkuliert. Die Fluidflussrate wird aus den gemessenen Konzentrationen errechnet.

Mit Hilfe eines Hematokrit-Sensors ist es ebenfalls möglich Rezirkulation mit Hilfe eines Bolus auf der Blutseite zu bestimmen.

Sämtliche genannten Verfahren lassen zwar grundsätzlich qualitative und quantitative Aussagen zur Rezirkulation im vaskulären Zugang des Dialysepatienten zu, jedoch haben die Verfahren des Standes der Technik eine Reihe von Nachteilen:

Die Temperaturdilutionsmethode ist anfällig für äußere Einflüsse wie Umgebungstemperatur. Da zwischen Messort und Patient eine Schlauchstrecke vorliegt, kann an diesen Stellen durch Energieverluste oder Energieeintrag die Messung verfälscht werden. Eine weitere Verfälschung kann durch die Wechselwirkung des thermischen Bolus mit der Temperatur des Patienten zustande kommen, da der Bolus den Shunt und den kardiopulmonalen Kreislauf passieren muss. Außerdem sind in der Regel zwei Sensormodule und damit ein hoher apparativer Aufwand nötig, um arteriell und venös Messungen vorzunehmen, was Dialysegeräte, die dieses Verfahren einsetzen, verteuert.

Die oben beschriebene Blutstromumkehr während der laufenden Dialyse ist medizinisch betrachtet wegen des zusätzlichen Fehlerrisikos einer versehentlichen Vertauschung der arteriellen und venösen Leitungen im Routinebetrieb kaum empfehlenswert.

Eine Erfassung der Rezirkulation mittels Dopplersonographie ist apparativ extrem aufwendig und teuer und zusätzlich muss manuell ein Kochsalzbolus gegeben werden.

Der Nachteil der manuellen Bolusgabe gilt auch für die Hämatokriterfassung mit einem externen Gerät, welches in der Anschaffung zudem sehr teuer ist.

Ausgehend vom Stand der Technik der Bolus-Verfahren zur Bestimmung der Rezirkulation ist es somit Aufgabe der vorliegenden Erfindung Verfahren und Vorrichtung für eine vollautomatische Messung einer Zugangsrezirkulation bei einem dialysepflichtigen Patienten zur Verfügung zu stellen. Diese Aufgabe wird durch ein Verfahren mit den kennzeichnenden Merkmalen der Ansprüche 1 oder 2 gelöst.

Vorrichtungstechnisch wird die Aufgabe durch eine Vorrichtung mit den kennzeichnenden Merkmalen der Ansprüche 7 oder 8 gelöst.

Die Erfindung wird durch die Merkmale der unabhängigen Ansprüche 1, 2, 7, 8 definiert.

Insbesondere betrifft die vorliegende Erfindung ein Dialysemaschine-Steuerungsverfahren zur maschinenseitigen (nicht patientenseitigen) Erfassung einer Rezirkulation in einem arteriovenösen Shunt eines Patienten vorzugsweise während einer laufenden Hämodialyse mittels der Dialysemaschine selbst, wobei die Dialysemaschine oder das -system eine patientenabgewandte Seite (Dialysierflüssigkeit-Seite) und eine patientenzugewandte Seite (Blut-Seite) aufweist;

wobei auf der Dialysierflüssigkeit-Seite des Dialysesystems wenigstens ein Dialysierflüssigkeitseinlass sowie wenigstens ein Dialysierflüssigkeitsabfluss für eine ausgewählte Dialysierflüssigkeit und wenigstens eine Fluid-/Dialysierflüssigkeitspumpe vorgesehen sind, welche in fluidischer Verbindung mit wenigstens einem Dialysator stehen, welcher eine semipermeable Membran aufweist, welche die Grenze zwischen Dialysierflüssigkeit- und Blut-Seite bildet; und wobei die Dialysierflüssigkeit auf der Dialysierflüssigkeit-Seite der Membran eine Dialysierflüssigkeitskammer des Dialysators in einer vorgegebenen Richtung durchströmt;

wobei auf der Blut-Seite des Dialysesystems insbesondere in einem extrakorporalen Kreislaufzweig eine Blutpumpe vorgesehen wird, die dafür angepasst ist, Blut durch eine Blutkammer des Dialysators zu führen, um urämische Toxine durch Diffusion über die semipermeable Membran aus dem extrakorporal geführten Blut zu entfernen;

wobei

ein Sensor zum Erfassen einer Änderung eines physikalisch-chemischen Parameters $P_D$ der abfließenden verbrauchten Dialysierflüssigkeit in der distalen Fluidik vorgesehen wird; und Steuerelemente derart in der distalen Fluidik angeordnet werden, dass die Dialysierflüssigkeit wahlweise vor Eintritt in die Dialysatorkammer unverändert durch den/am Sensor entlang geführt wird; wodurch der Sensor mit unverbrauchter (reiner) Dialysierflüssigkeit kalibriert wird;

und wobei

die Steuerelemente nach der Kalibration so eingestellt werden, dass die Dialysierflüssigkeit die Dialysatorkammer durchströmt und im blutseitigen extrakorporalen Kreislaufzweig ein gewünschter erster Blutflusswert $BF_1$ eingestellt wird, wodurch sich zwischen einem Eingang des extrakorporalen Kreislaufzweiges und einem Ausgang des extrakorporalen Kreislaufzweiges eine Rezirkulation R einstellt und der vom Sensor (6) erfasste Parameter einen Wert $P_{D1}$ annimmt;

der erste Blutflusswert $BF_1$ auf einen zweiten Blutflusswert $BF_2$ geändert wird, wobei am Sensor ein neuer Parameter-Wert $P_{D2}$ vorliegt und erfasst wird;

und der Änderungsverlauf (oder die Änderung) von $P_{D1}$ zu $P_{D2}$ verwendet wird, um in Abhängigkeit der Art und Weise des Änderungsverlaufs (der Änderung) die Rezirkulation zu erfassen oder zu bestimmen. Dabei sei darauf hingewiesen, dass die Rezirkulation für zumindest einen aus dem ersten und zweiten Blutflusswert Null bzw. vernachlässigbar oder zumindest vorbekannt ist.

Des Weiteren betrifft die vorliegende Erfindung eine Dialysemaschine bzw. ein Dialysesystem mit einer Einrichtung zur Erfassung einer Rezirkulation in einem Bluteinlass-/Auslasselement vorzugsweise arteriovenösen Shunt eines Patienten während einer laufenden Hämodialyse, wobei das Dialysesystem eine patientenabgewandte Seite (Dialysierflüssigkeit-Seite) und eine patientenzugewandte Seite (Blut-Seite) aufweist;

wobei auf der Dialysierflüssigkeit-Seite des Dialysesystems wenigstens ein Dialysierflüssigkeiteinlass sowie wenigstens ein Dialysierflüssigkeitabfluss für eine ausgewählte Dialysierflüssigkeit und wenigstens eine Dialysierflüssigkeitspumpe vorgesehen sind, welche in fluidischer Verbindung mit wenigstens einem Dialysator steht, der eine semipermeable Membran aufweist, welche die Grenze zwischen Dialysierflüssigkeit- und Blut-Seite bildet; und wobei die Dialysierflüssigkeit auf der Dialysierflüssigkeit-Seite der Membran eine Dialysierflüssigkeitskammer des Dialysators in einer vorgegebenen Richtung durchströmt;

wobei auf der Blut-Seite des Dialysesystems Blut in einem extrakorporalen Kreislaufzweig mittels einer Blutpumpe vorzugsweise in Gegenrichtung zur Strömungsrichtung der Dialysierflüssigkeit durch eine Blutkammer führbar ist, um urämische Toxine insbesondere durch Diffusion über die semipermeable Membran aus dem extrakorporal geführten Blut zu entfernen;

wobei

wenigstens ein Sensor zum Erfassen einer Änderung oder Änderungsverlaufs eines physikalisch-chemischen Parameters $P_D$ (z.B. Absorption, Absorbanz, etc.) der abfließenden, verbrauchten Dialysierflüssigkeit in der distalen Fluidik vorgesehen ist; und vorzugsweise Steuereinrichtungen derart in der distalen Fluidik angeordnet sind, dass die Dialysierflüssigkeit in ausgewählter Weise vor Eintritt in die Dialysatorkammer unverändert durch den/längs des Sensor(s) fließt; um weiter vorzugsweise eine Kalibration des Sensors mit reiner/unverbrauchter Dialysierflüssigkeit vorzunehmen;

die Steuereinrichtungen nach der (optionalen) Kalibration so einstellbar/eingestellt sind, dass die Dialysierflüssigkeit die Dialysatorkammer durchströmt und im proximalen extrakorporalen Kreislaufzweig ein gewünschter erster Blutflusswert $BF_1$ einstellbar/eingestellt ist, wodurch sich zwischen einem Eingang des extrakorporalen Kreislaufzweiges und einem Ausgang des extrakorporalen Kreislaufzweiges eine Rezirkulation R einstellt und der vom Sensor erfasste Parameter einen Wert $P_{D1}$ aufweist;

der erste Blutflusswert $BF_1$ auf einen zweiten Blutflusswert $BF_2$ (vorzugsweise mittels Blutpumpe) änderbar ist (beispielsweise sprungartig, rampenartig, etc.), wobei am Sensor ein neuer zweiter Parameter-Wert $P_{D2}$ vorliegt; und

Mittel vorgesehen sind, um den Änderungsverlauf und/oder die Änderung von $P_{D1}$ zu $P_{D2}$ zu erfassen, und daraus die Rezirkulation R zu bestimmen.

Die Unteransprüche geben bevorzugte Ausführungsformen der vorliegenden Erfindung wieder.

Ein bevorzugtes Verfahren ist ein solches, bei welchem als physikalisch-chemischer Parameter $P_D$ eine optische Absorption, insbesondere eine Absorption im IR-Wellenlängenbereich, UV-Wellenlängenbereich oder im sichtbaren Wellenlängenbereich oder eine entsprechende Absorbanz verwendet wird.

Für die Praxis hat sich als vorteilhaft herausgestellt, als physikalisch-chemischen Parameter $P_D$ die UV-Absorption und/oder die UV-Absorbanz zu verwenden. Dabei werden insbesondere LEDs eingesetzt, die UV-Licht bei einer Wellenlänge von ca. 280 nm emittieren. Zweckmäßigerweise wird zur quantitativen Erfassung der Absorption (Absorbanz) ein UV-Detektor als Sensor verwendet. Der Vorteil des eingesetzten Wellenlängenbereiches liegt darin, dass harnpflichtige Substanzen in diesem Bereich gute Absorptionen aufweisen, womit eine große Sensivität unter Einsatz von Standardbauteilen erreicht werden kann.

In einer bevorzugten Ausführungsform des erfindungsgemäßen Verfahrens ist der zweite Blutflusswert $BF_2$ (vorzugsweise erheblich) kleiner als der erste Blutflusswert $BF_1$, wobei insbesondere gilt: $BF_2 = r \times BF_1$, vorzugsweise mit $r = 0{,}05$ bis $0{,}95$. D. h. der Wert $BF_2$ kann deutlich oder auch nur minimal kleiner sein als der Wert $BF_1$ und zwar weiter vorzugsweise in Abhängigkeit vom Arbeitsbereich der aktuell eingesetzten Blutpumpe.

Durch eine derartige Bedingung kann der Blutfluss auf einen Wert eingestellt werden, der unter dem natürlichen Shuntfluss liegt. In einem solchen Fall liegt die Rezirkulation im Shunt in sehr guter Näherung bei Null, so dass sich bei Vorliegen einer Rezirkulation bei dem zur Therapie eingestellten Dialyse-Blutflusswert eine gut detektierbare Änderung für den physikalisch-chemischen Parameter ergibt.

Es kann jedoch auch von Vorteil sein, den umgekehrten Weg zu beschreiten, bei welchem der zweite Blutflusswert $BF_2$ (vorzugsweise erheblich) größer ist als der erste Blutflusswert $BF_1$ wobei insbesondere gilt: $BF_2$ = r x $BF_1$, vorzugsweise mit r = 1,05 bis 20 und zwar ebenfalls vorzugsweise in Abhängigkeit der aktuell verwendeten Blutpumpe. In diesem Fall läge der bevorzugte Arbeitsbereich der Blutpumpe (wie auch bei der vorstehenden Angabe) zwischen 30ml/min und 600ml/min. An dieser Stelle sei aber ausdrücklich darauf hingewiesen, dass der genannte Arbeitsbereich der Blutpumpe auch auf einen anderen Wert festgelegt sein kann, wobei sich dann in Abhängigkeit hiervon die Differenz zwischen den beiden Blutflusswerten anders (d.h. außerhalb der vorstehend genannten Bereiche) bestimmen würde.

Eine weitere bevorzugte Ausführungsform des erfindungsgemäßen Verfahrens ist dadurch gekennzeichnet, dass die Rezirkulation R durch ein Einschwingverhalten des zeitlichen Verlaufs der Änderung des physikalisch-chemischen Parameters $P_D$ nach Änderung des ersten Blutflusses $BF_1$ auf den zweiten Blutfluss $BF_2$ erfasst wird.

Durch eine derartige Transientenanalyse wird erreicht, dass die vorliegende Erfindung zuverlässige quantitative und/oder qualitative Daten der Rezirkulation während der Dialyse liefert, ohne externe Maßnahmen wie Bolusinjektionen zu benötigen.

Eine bevorzugte Durchführung der Transientenanalyse erfolgt durch ein Verfahren, bei welchem das Einschwingverhalten durch eine Dämpfung δ erfasst wird und mittels der Dämpfung δ (Delta) des Einschwingverhaltens nach Anlegen des zweiten Blutflusses $BF_2$ die Rezirkulation R über einen entsprechenden Algorithmus, der in den Beispielen näher erläutert wird, bestimmt wird.

Eine alternative Durchführung der Transientenanalyse kann dadurch erfolgen, dass das Einschwingverhalten nach Anlegen des zweiten Blutflusses $BF_2$ durch eine Integration eines normierten Zeitsignals, insbesondere einer vom Sensor ausgegebenen Intensität, über einen bestimmten Zeitbereich, erfasst wird und hiermit die Rezirkulation R über einen entsprechenden Algorithmus, der in den Beispielen näher erläutert wird, bestimmt wird.

Eine weitere alternative Durchführung der Transientenanalyse kann dadurch erfolgen, dass das Einschwingverhalten nach Anlegen des zweiten Blutflusses $BF_2$ durch eine charakteristische Zeit T eines Signalanstiegs des vom Sensor gelieferten Ausgangssignals erfasst wird und hiermit die Rezirkulation R über einen entsprechenden Algorithmus, der in den Beispielen näher erläutert wird, bestimmt wird.

Ein besonderer Vorteil der unterschiedlichen Verfahren der Transientenanalyse liegt darin begründet, dass die Ergebnisse wenigstens zweier unterschiedlicher Verfahren zur Mittelwertbildung verwendet werden, um Plausibilitätsprüfungen durchzuführen und/oder die Genauigkeit zu erhöhen.

Zur Überwachung der Druckverhältnisse, dem laufenden Betrieb und dem korrekten Anschluss des Bluteinlass-/Auslasselements, vorzugsweise Shunts an die proximale Seite des Dialysesystems können in dem Dialysesystem Drucksensoren im proximalen Kreislauf für den arteriellen Druck (PA), den venösen Druck (PV) und den Eingangsdruck (Pressure before Entrance [PBE]) für den Dialysator vorgesehen sein. Sofern eine Auswertung der Drucksensoren vorprogrammierte Schwellwerte über- oder unterschreitet, werden entsprechend vorprogrammierte Steuerungs- und Regelungsmaßnahmen eingeleitet.

Mit Hilfe der vorliegenden Erfindung ist es möglich, eine Messung zur Bestimmung der Rezirkulation im Shunt durchzuführen, ohne die Blutseite durch einen Bolus zu beeinflussen. Somit ist es möglich, punktuelle Messungen durchzuführen, ohne dass zusätzliche Messgeräte dafür benötigt werden.

Verfahren und Vorrichtung gemäß der vorliegenden Erfindung weisen daher folgende Vorteile auf:

- Es kann online die Situation am Patientenzugang überwacht werden
- es muss keine Bolusinjektion verwendet werden
- es wird ein Transientensignal nach Systemzustandsänderung analysiert, dass mit Hilfe eines Sensors auf der Dialysatseite gemessen wird
- vorzugsweise kann ein optischer, insbesondere ein UV-Sensor, verwendet werden
- die Erfindung ermöglicht eine kontinuierliche Überwachung der Shuntsituation durch Speichern und Trendauswertung der Messungen
- es kann eine automatische Rezirkulationserkennung nach getriggerter Messung während der laufenden Therapie durchgeführt werden
- es liegen kurze Messzeit von unter 4 min vor
- Verfahren und Vorrichtung sind kostengünstig, da bereits eine ohnehin vorhandene Sensorik verwendet werden kann
- geringer apparativer Aufwand
- Messung wird auf der Dialysierflüssigkeitsseite durchgeführt, so dass keinerlei Interaktion des Sensors mit dem Blut des Patienten stattfindet, so dass keine mikrobiologische Kontamination zu befürchten ist
- durch Messung auf der Dialysierflüssigkeitsseite hat Pflegepersonal keinen zusätzlichen Aufwand bei Vorbereitung des Dialysesystems und Einlegen des Blutschlauchsystems in die Sensoren
- Messung bedarf keines personalintensiven Schrittes.

Weitere Vorteile und Merkmale der vorliegenden Erfindung ergeben sich aufgrund der Beschreibung von Ausführungsbeispielen sowie anhand der Zeichnung.

Es zeigt:

Fig. 1　eine schematische Darstellung der Fluidik eines beispielhaften Dialysesystems gemäß der vorliegenden Erfindung;

Fig. 2     eine schematische Darstellung der Fluidik einer proximalen Seite eines Dialysesystems mit Rezirkulation im Shunt während der Therapie;

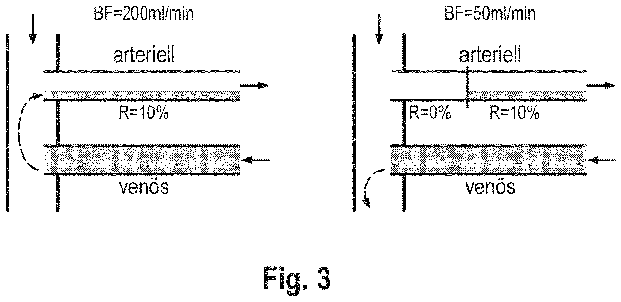

Fig. 3     eine schematische Darstellung der Rezirkulation bei unterschiedlichen Blutflüssen;

Fig. 4     ein Diagramm eines Einschwingvorganges eines erfassten Signals mit und ohne Rezirkulation (von Level 0 zu Level 2);

Fig. 5     ein Diagramm für die Korrelation zwischen Dämpfung $\delta$ und der Rezirkulation im Shunt;

Fig. 6     ein Diagramm für die Korrelation der Rezirkulation und (1-$\delta$);

Fig. 7     Dialysator-Rezirkulationskennlinien für unterschiedliche Dialysatoren;

Fig. 8     ein Diagramm für die Korrelation der Rezirkulation und (1-$\delta$) bei unterschiedlichen Blütflüssen für einen ausgewählten Dialysator;

Fig. 9     eine Diagrammdarstellung des bestimmten Integrals des normierten Zeitsignals;

Fig. 10     eine Diagrammdarstellung der Korrelation zwischen Integral und Rezirkulation für unterschiedliche Dialysatoren;

Fig. 11     eine Diagrammdarstellung der Abhängigkeit des Integrals vom Blutfluss;

Fig. 12     eine Diagrammdarstellung der charakteristischen Zeit T eines Signalanstiegs;

Fig. 13     eine Diagrammdarstellung der normierten Intensitätssignale und der Zeitkonstanten T bei unterschiedlichen Rezirkulationsniveaus;

Fig. 14     eine Diagrammdarstellung der Abhängigkeit der Zeitkonstante T vom eingestzten Dialysator;

Fig. 15     eine Diagrammdarstellung der Abhängigkeit der Zeitkonstante T vom eingestellten Blutfluss;

Fig. 16     ein Clearance - Diagramm im Fall einer geringen Blutflussänderung, diagrammatisch den Zusammenhang von effektiver Clearance versus Dialysatorclearance im Modell berechnet;

Fig. 17     diagrammatisch den Zusammenhang von effektiver Clearance versus Dialysatorclearance gemessen, und

Fig. 18     diagrammtechnische Darstellung eines Beispiels einer Absorptionsänderung bei geringer Blutflussänderung.

Das Ziel dieser Erfindung ist es, eine Messung ohne eine Bolusgabe zu ermöglichen. Parallel dazu soll das Ganze ohne zusätzlichen technischen Aufwand erfolgen und somit auf der Sensorik basieren, die bereits in einem gattungsgemäßen Dialysegerät (ohnehin) vorhanden ist.

In dem Flussdiagramm der Fig. 1 ist in schematischer Darstellung ein erfindungsgemäßes Dialysesystem mit einer patientenabgewandten Seite (Dialysierflüssigkeit-Seite) und einer patientenzugewandten Seite (Blut-Seite) gezeigt.

Mit 1 ist ein Dialyseflüssigkeitseinlass und mit 2 ein Dialyseflüssigkeitsablass bezeichnet. Über eine Flusspumpe 3 wird Dialysierflüssigkeit zu einer Dialysierflüssigkeitskammer 4a eines Dialysators 4 gepumpt, so dass die Dialyseflüssigkeit die Kammer 4a in einer vorgegebenen Richtung durchströmt. Der Dialysator 4 weist eine in Fig. 1 nicht gezeigte semipermeable Membran auf, die die Grenze zwischen distaler und proximaler Seite des Dialysators 4 bildet. Dialysator 4 weist ferner auf der proximalen Seite eine Blutkammer 4b auf.

Am Dialyseflüssigkeitsablass 2 ist ein UV-Detektor als Sensor 6 angeordnet. Zur Kalibrierung des UV-Sensors 6 mit reiner Dialyseflüssigkeit sind stromauf und stromab zu der Dialyseflüssigkeitskammer 4a Ventile 8 und 9 als Steuereinrichtungen/-elemente angeordnet, welche derart einstellbar sind, dass reine Dialyseflüssigkeit über einen durch die Ventile 8 und 9 gebildeten Bypass fließt und nicht durch Dialysatorkammer 4a.

Auf der Blut-Seite des Dialysesystems wird zu reinigendes Blut aus einem arteriovenösen Shunt eines Patienten aus einem Eingang 20 des extrakorporalen Kreislaufzweiges mittels einer Blutpumpe 5 und einem voreingestellten Blutfluss, im Beispielsfalle vorzugsweise 200 ml/min, durch die Blutkammer 4b weiter vorzugsweise im Gegenstrom zur Dialyseflüssigkeit gepumpt, wobei sich über die semipermeable Membran eine definierte erste Clearance der membrangängigen harnpflichtigen Substanzen einstellt und hierdurch die natürliche Nierenfunktion weitgehend nachgeahmt wird und somit das (arterielle) Blut des Patienten gereinigt wird. Aus der Blutkammer 4b fließt gereinigtes (venöses) Blut über einen Ausgang 21 des extrakorporalen Kreislaufs zum Patienten zurück. Eine typische Hämodialyse dauert mehrere Stunden. Zur Überwachung und Regelung des gesamten Dialyseverfahrens sind blutseitig ein Sensor PV für den venösen Druck, ein Sensor PA für den arteriellen Druck sowie ein Sensor PBE (Pressure Before Entrance) für den Druck am Eingang der Blutkammer 4b angeordnet.

### 1. Das UV-Messverfahren

Das UV-Messverfahren selbst ist in der deutschen Patentschrift DE 699 16 053 T2 (Althin Medical AB) beschrieben, auf die hiermit diesbezüglich vollinhaltlich Bezug genommen wird. Jedoch wird im Folgenden das UV-Messverfahren, welches im Rahmen der vorliegenden Erfindung zum Einsatz kommt, kurz beschrieben:

Um eine angemessene Dialysebehandlung zu gewährleisten, wurde gemäß DE 699 16 053 T2 das sogenannte Kt/V (Harnstoffmodell) entwickelt, wobei K für die Fähigkeit des Dialysators steht, Harnstoff in ml/min aus dem Blut zu entfernen, t für die Behandlungsdauer in Minuten steht und V die Verteilung des Harnstoffs im Körper in ml bezeichnet, die auf das Körpergewicht des Patienten bezogen ist. Der dimensionslose Faktor Kt/V (Harnstoff) definiert die Ausscheidung des Harnstoff-Stickstoffgehaltes im Blut und kann beispielsweise bei drei Behandlungen pro Woche größer oder gleich 1 sein.

Das Messprinzip des UV-Sensors zur Bestimmung des Kt/V aus der oben genannten Patentschrift beruht auf dem Prinzip der Fotometrie.

Die Lichtquelle besteht aus einer LED und als Detektoren dienen beispielsweise zwei Fotodetektoren. Die LED emittiert ein Signal mit einer Wellenlänge von ca. 280nm. Diese Wellenlänge wird von harnpflichtigen Subtanzen absorbiert.

Der Sensor 6 befindet sich im Dialysatabfluss 2 hinter dem Dialysator 4 und misst während der Therapie die Absorption A (Intensität) im

$$A = \log_{10}\left(\frac{Detektor_0}{Detektor_i}\right)$$

Dialyseflüssigkeitsabfluss 2 kontinuierlich. Zu Beginn wird der Detektor$_0$ Wert mit reiner Dialyseflüssigkeit zur Kalibration ermittelt.

Der Detektor$_0$ Wert ist dabei das zu Beginn kalibrierte Level, das mit einer Subtanz, in der Regel die unverbrauchte Dialyseflüssigkeit, ohne die zu messenden (toxischen) Subtanzen durchgeführt wird. Daher wird die Kalibrierung vor dem Anlegen des Patienten bzw. im Bypass über Ventile 8 und 9 durchgeführt.

Der Dialyseflüssigkeitsfluss , der Blutfluss BF und der eingesetzte Dialysator 4 beeinflussen die Absorption und somit die vorherrschende Clearance, die maßgeblich für die Absorption im Dialyseflüssigkeitsabfluss 2 ist. Dabei werden die entzogenen Giftstoffe von der Blutseite (Clearance) durch den Dialyseflüssigkeitsfluss verdünnt und im Dialyseflüssigkeitsabfluss 2 die Absorption gemessen. Der UV-Sensor 6 und die Drucksensoren PV, PA und PBE sind wie in Fig. 1 gezeigt eingeschlaucht.

## 2. Detektion einer Rezirkulation im Shunt

Sofern eine Rezirkulation im Shunt auftritt, wird schon gereinigtes Blut aus dem venösen Zugang anteilig wieder in den arteriellen Zugang übergeben. Die Rezirkulation R in [%] ist das Verhältnis zwischen gereinigtem Blut und dem eigentlichen Blutfluss. Dadurch wird nicht die eigentliche im Patienten vorhandene Konzentration an Giftstoffen zum Dialysator befördert sondern nur eine Verdünnung mit bereits gereinigtem Blut, wodurch sich die Dialyseeffektivität verringert.

In Fig. 2 ist in schematischer Darstellung eine Rezirkulation in einem Shunt während einer Dialysetherapie gezeigt. Dabei gilt

$$Rezirkulation\ R\ [\%] = \frac{Q_R}{Q_B}$$

$$Q_B = Q_R + Q_P$$

wobei $Q_R$ der Fluss des rezirkulierenden Blutes ist, $Q_B$ der Blutfluss im extrakorporalen Kreislauf ist, also dem an Blutpumpe 5 eingestellten Fluss/Flow entspricht und $Q_P$ der Blutfluss im Shunt des Patienten ist.

## 3. Verfahren zur Bestimmung einer Rezirkulation im Shunt durch eine Blutflussänderung und Auswertung des Einschwingvorgangs

Um eine Rezirkulation im Shunt zu messen, besteht das Verfahren aus verschiedenen Intervallen, die es ermöglichen, die Bestimmung ohne externe Einflüsse (insbesondere auf der Patientenseite) durchzuführen.

Rezirkulation bedeutet dabei der Anteil im extrakorporalen Blutkreislauf, der bereits gereinigt wieder aus den venösen Zweig aufgenommen/zurückgeführt wird und so die effektive Dialyseleistung reduziert. Der Ansatz beruht darauf, dass eine durch eine Verringerung des Blutflusses die Rezirkulation ebenso verringert wird. Sofern der Blutfluss kleiner oder gleich dem Shuntfluss ist, tritt idealerweise keine Rezirkulation mehr auf.

Im Rahmen der vorliegenden Erfindung wird dieser Hintergrund verwendet, um (lediglich) durch die Änderung des Blutflusses den Anteil der Rezirkulation festzustellen.

Der eingestellte Dialyse-Blutfluss in der Therapie hat somit je nach Shuntfluss eine konstante Rezirkulation. Dadurch befindet sich eine gegenüber der Konzentration im Blut des Patienten verdünnte Konzentration urämischer Toxine im extrakorporalen Blut mit einer verdünnten Konzentration bezogen auf die eigentliche Konzentration im intrakorporalen Blutkreislauf des Patienten.

Durch eine Änderung des Blutflusses auf einen Wert kleiner (z.B. 50ml/min) oder gleich dem Shuntfluss wird die Clearance am Dialysator verändert.

[vgl. KF KDOQI Guidelines. Guideline 3. Methods for postdialysis blood sampling. [Internet]. [cited 2009 Aug 18]; Available from:

http://www.kidney.org/professionals/KDOQI/guideline_upHD_PD_VA/hd_guide3.htm]

Dadurch schwingt die Konzentration urämischer Toxine am Ausgang des Dialysators 4 auf ein neues Level ein, das abhängig von der sich neu einstellenden Clearance ist. Sofern bei einem ersten (bestimmten/eingestellten) Blutfluss BF$_1$ der Therapie eine Rezirkulation bereits vorhanden ist, befindet sich Blut mit einer Konzentration an urämischen Toxinen C1 im arteriellen Zugang 20 des Schlauchsystems, wobei im Patienten das Blut eine Konzentration an urämischen Toxinen von C0 hat. Die Differenz der beiden Konzentrationen C1 und C0 wird durch die Rezirkulation verursacht.

Bei einer Änderung auf einen beispielsweise geringeren zweiten (bestimmten) Blutfluss $BF_2$ der angenommener Weise kleiner als der Shuntfluss sei, existiert keine Rezirkulation mehr. Dadurch hat nach kurzer Zeit das in das Schlauchsystem (extrakorporaler Blutkreislauf) aufgenommene Blut wieder die Konzentration C0 entsprechend dem Blut im Patienten. Nach der Änderung des Blutflusses ist jedoch noch Blut mit der Konzentration C1 im Schlauchsystem und wird nun mit dem neuen zweiten Blutfluss $BF_2$ und so auch der neuen Clearance $K_2$ gereinigt.

Sofern zuvor keine Rezirkulation bestanden hat, ist C1 = C0. Der Einschwingvorgang erfolgt in diesem Fall ohne Überschwingen auf das neue Level L2.

Sofern der erste Blutfluss $BF_1$ > Shuntfluss ist, zeigt der Einschwingvorgang jedoch ein Überschwingen, da eine Rezirkulation vorhanden ist. Die Intensität dieses Überschwingvorgangs ist damit direkt proportional zur Rezirkulation.

### 3.1 Die mathematische Bestimmung der Rezirkulation mittels einer Transientenmethode nach Blutflussänderung über die Dämpfung $\delta$ (Delta)

Durch die Bestimmung der Übertragungsfunktion aus dem aufgenommenen Kurvenverlauf werden die Parameter der Funktion F(s) ermittelt. Diese Charakterisierung des Kurvenverlaufs bildet die Grundlage zur Berechnung der Rezirkulation.

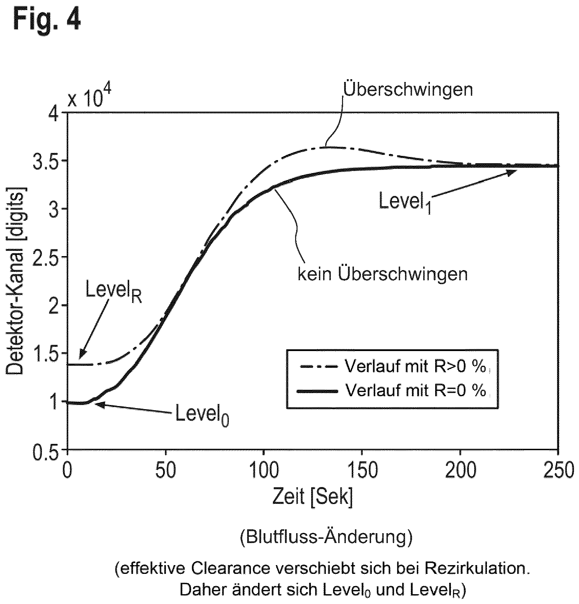

Mögliche Kurvenverläufe sind in Fig. 4 abgebildet, die jeweils den Einschwingvorgang mit und ohne Rezirkulation darstellen. Die Blutkonzentration ist dabei in beiden Fällen mit C0 gleich. Das unterschiedliche Level zu Beginn ist ebenfalls direkt proportional zur Rezirkulation, da aus diesem Grund die Konzentration C1 im Schlauchsystem sich unterscheidet und auf Grund der gleichen Dialysator - Clearance direkt übertragen wird. Bei beiden Messungen existiert auf Grund eines gleichen Blutflusses BF gemäß der Fig. 4 für das Level 0 und das Level R die gleiche Dialysator - Clearance. Diese bildet die Übertragungsfunktion zwischen der Blut- und der Dialysierflüssigkeitsseite.

$$C_B \cdot K = C_D \cdot Q_D$$

$$A_{D\_i} = \log \frac{Det_0}{Det_i}$$

$$A_{D\_i} \sim C_{D\_i}$$

Auf Grund der Rezirkulation im Shunt ist die Konzentration im extrakorporalen Blutschlauchsystem C1 < C0 und somit ebenfalls das Detektorsignal Level R höher, da weniger Substanzen bei gleicher Clearance $K_1$ entzogen werden. Sofern keine Rezirkulation existiert ist C1 = C0.

Die Charakterisierung der Übertragungsfunktion kann durch die folgenden Gleichung allgemein beschrieben werden.

$$F(s) = \frac{N(s)}{P(s)}$$

$$F(s) = \frac{s^i + n_1 \cdot s^{i-1} + \ldots + n_{i-1} \cdot s + n_i}{p_0 \cdot s^j + p_1 \cdot s^{i-1} + \ldots + p_{j-1} \cdot s + p_j}$$

Beispielhaft kann die Sprungantwort einer Übertragungsfunktion, die als PT3 Regelstrecke bekannt ist, zur Chararakterisierung des Vorgangs gewählt werden. Die PT3 Regelstrecke hat die folgende Form:

$$F(s) = \left( \frac{1}{T^2 \cdot s^2 + 2 \cdot \delta \cdot T \cdot s + 1} \right) \cdot \left( \frac{1}{s + c} \right)$$

Wobei der Operator s für den Ableitungsoperator $s = \dfrac{d}{dt}$ steht.

Eine allgemeine Berechnung ist durch Bestimmung der Polstellen möglich. Dazu werden aus der zuvor bestimmten Übertragungsfunktion die Polstellen bestimmt

$$Polpaar_{konjugiert\_konplex} = -\frac{\delta}{T} \pm \sqrt{\left(\frac{\delta}{T}\right)^2 - \frac{1}{T^2}}$$

$$a = \frac{\delta}{T}$$

$$b = \sqrt{\left(\frac{\delta}{T}\right)^2 - \frac{1}{T^2}}$$

$$F(s) = \left( \frac{1}{(s+a+bj)\cdot(s+a-bj)\cdot(s+c)} \right)$$

und mit Hilfe des komplexen Polpaars erfolgt die Berechnung der Dämpfung $\delta$.

$$\delta = \left| \left( \frac{\mathrm{Re}\{Polpaar\}}{|Polpaar|} \right) \right|$$

$$\delta = \left| \left( \frac{a}{\sqrt{(a^2+b^2)}} \right) \right|$$

Neben dem Einschwingverhalten durch die Volumen im Dialysator 4 beschreibt der Parameter $\delta$ die Dämpfung.

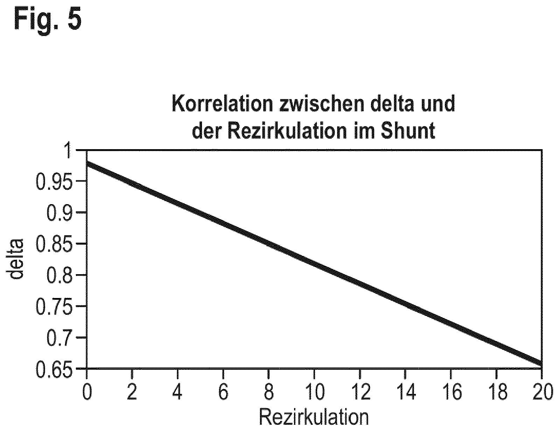

Sofern das System bei der Blutflussänderung im Schlauchsystem (extrakorporaler Blutkreis) die Blutkonzentration von $C_R = C_0$ hat, ist der Parameter $\delta \sim 1$. Dabei sei erwähnt, dass gemäß der Fig. 5 die Dämpfung für den rezirkulationsfreien Fall abhängig vom verwendeten Dialysator auch (geringfügig) kleiner oder größer 1 sein kann.

Bei einer vorhandenen Rezirkulation und so $C_1 < C_0$ schwingt das System über und somit wird $\delta$ proportional zur Rezirkulation kleiner. Die Bestimmung von R ist somit direkt aus der bestimmten Dämpfung $\delta$ möglich und wird über die folgende Korrelation bestimmt.

Die Steigung der Funktion ist dabei abhängig vom Blutfluss des einschwingenden Levels und somit ebenfalls von der Clearance. Damit ergibt sich für jeden Blutfluss eine Funktion, mit der die Rezirkulation R bestimmt wird.

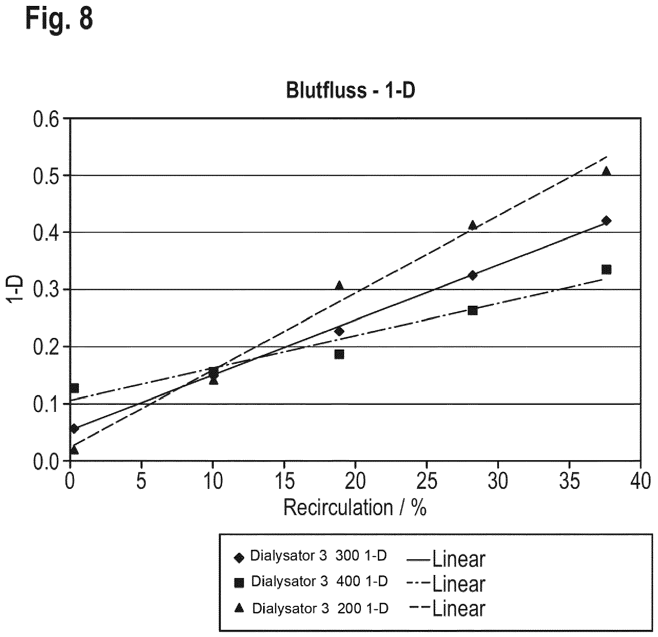

Um gemäß der Fig. 8 einen Anstieg > 0 zu erhalten, wurde 1- $\delta$ = 1-D auf der y-Achse aufgetragen. Fig. 4 zeigt ferner den erläuterten Einschwingvorgang des Signals mit und ohne Rezirkulation. Die Korrelation zwischen der Dämpfung $\delta$ und der Rezirkulation im Shunt ist in Fig. 5 gezeigt.

Die Einbeziehung des Blutflusses in den Auswertealgorithmus erlaubt die Wahl der entsprechenden Kennlinie und dadurch eine Erhöhung der Genauigkeit der Rezirkulationsbestimmung.

Die Übertragungsfunktion von dem bestimmten Wert der Dämpfung $\delta$ ist somit durch ein Polynom möglich, das beispielsweise erster Ordnung ist:

$$R = a \cdot (1 - \delta)$$

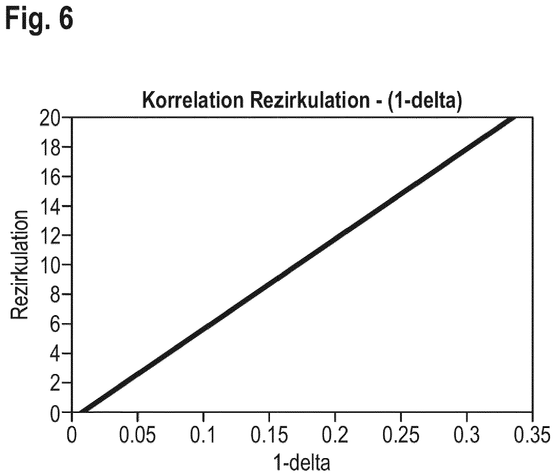

Die Korrelation der Rezirkulation R mit dem Term (1-$\delta$) ist in Fig. 6 gezeigt.

Das Einschwingverhalten nach Blutflussänderung ist abhängig vom jeweils verwendeten Dialysator 4, d.h. insbesondere vom Volumen des eingesetzten Dialysators 4, so dass sich für jeden Dialysator eine Kennlinie ergibt.

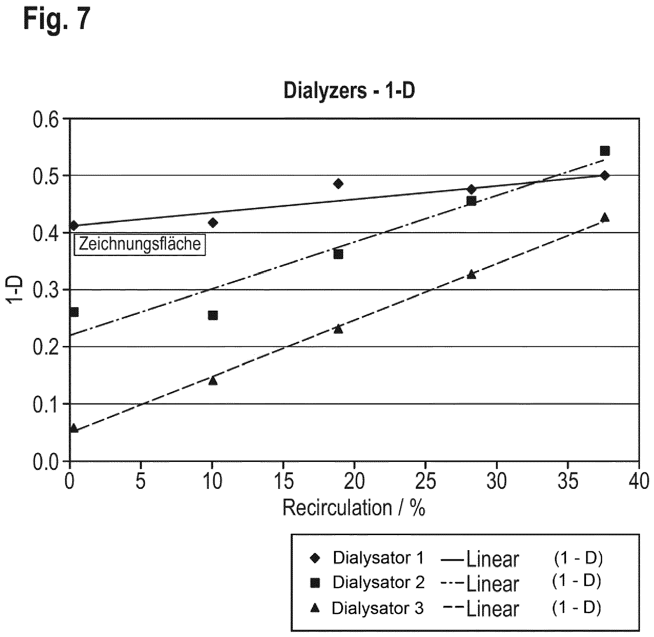

Eine solche Kennlinienschar ist in Fig. 7 gezeigt. Um einen positiven Anstieg zu erhalten, wurde in Fig. 7 auf der y-Achse 1-$\delta$ aufgetragen. Die Kenntnis des Dialysatortyps ist Vorraussetzung für eine genaue Bestimmung der Rezirkulation aus der Dämpfung der Übertragungsfunktion.

3.2 Die mathematische Bestimmung der Rezirkulation mittels einer Transientenmethode nach Blutflussänderung über die Integralmethode

Als auswertbarer Parameter kann auch das bestimmte Integral des normierten Zeitsignals (Intensität des UV-Sensors 6) herangezogen werden.

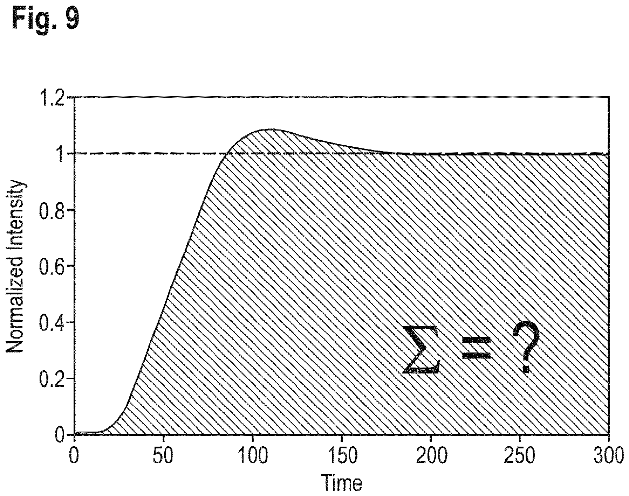

Hierbei wird gemäß Fig. 9 zwischen den definierten Zeiten $t_1$ und $t_2$ eine normierte Intensität, d.h. der 0-Wert des Signals vor der Änderung des Blutflusses, 1-Wert nach dem Einschwingvorgang auf das höhere Signallevel, aufsummiert.

Fig. 9 zeigt ein Beispiel bei dem:

$t_1$ = 0 s bezogen auf den Beginn der ausgelösten Blutflussänderung und
$t_2$ = 300 s bezogen auf den Beginn der ausgelösten Blutflussänderung ist.

Das Integral oder die Summe kann mit dem Fachmann wohlbekannten Methoden numerisch oder analytisch ermittelt werden, im Beispielsfalle:

$$"Größen\,variable..G" = \sum_{t=t_1}^{t=t_2} Detektor_t$$

mit $Detektor_t$ = gemessene Intensität im UV-Sensor 6 zum Zeitpunkt t.

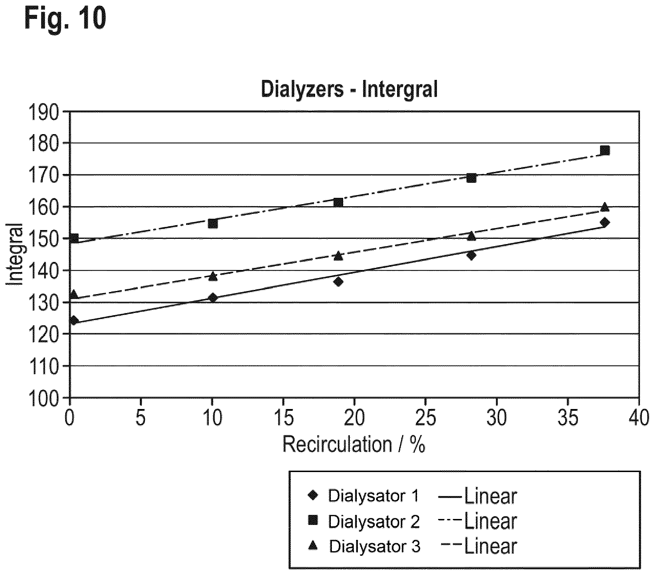

Auch der Transientenanalyse mittels der Integralmethode liegt wiederum das in Bezug auf die Dämpfung $\delta$ erläuterte Prinzip zugrunde, dass sich aus einer "Verdünnung" im arteriellen Schlauchsystem des proximalen Kreislaufzweigs und damit dem Abstellen (auch Anstellen in Abhängigkeit der Blutflussänderungsrichtung) einer vorhandenen Rezirkulation ein Überschwingen des verwendeten Zeitsignals ergibt. Das Prinzip ist in Fig. 4 illustriert. Der Wert, den die "Größenvariable" bei der Integralmethode annimmt, ist direkt proportional zum Rezirkulationsniveau, so dass sich eine Funktion für den Zusammenhang zwischen Rezirkulation R und Integral/Summe ergibt, die beispielsweise durch ein Polynom ersten Grades approximiert werden kann. Dieser Zusammenhang ist graphisch in Fig. 10 dargestellt. Auch hier ist die "Größenvariable" (in diesem Beispiel das Integral) abhängig vom verwendeten Dialysator, was ebenfalls in Fig. 10 verdeutlicht ist.

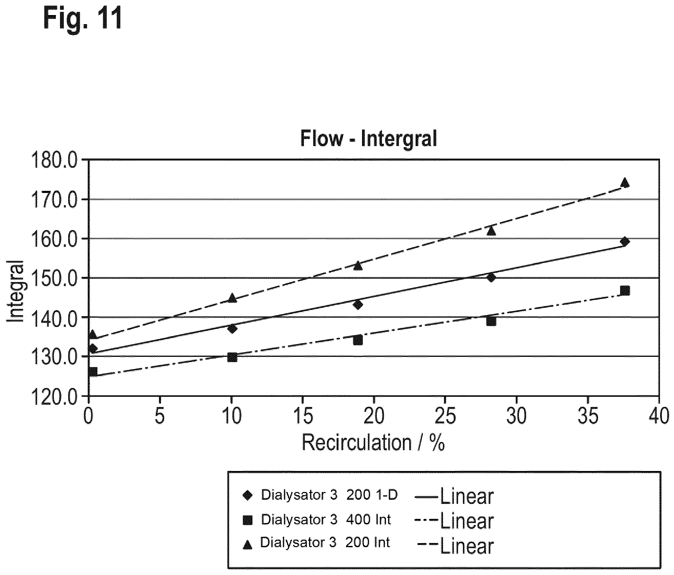

In Fig. 11 ist graphisch dargestellt, dass auch das Integral (wie auch die Dämpfung $\delta$) vom Blutfluss abhängig ist. Dies wirkt sich auf die Steigung und den Offset des funktionalen Zusammenhangs aus, der im Beispielsfalle durch ein Polynom ersten Grades approximiert wird. Auch im Falle der Integralmethode könnte durch einen empirisch ermittelten Korrekturfaktor die Messgenauigkeit durch Eingrenzung des blutseitigen Konzentrationsbereiches erhöht werden, wenn bei der Auswertung der gemessene Wert der Absorption bei BF = 50 ml/min zusätzlich herangezogen wird.

Für den Fachmann ist ersichtlich, dass die Kenntnis des eingesetzten Dialysators und des Blutflusses die Bestimmung der Rezirkulation deutlich verbessert, da damit der korrekte funktionale Zusammenhang zwischen der Größenvariablen (Integral) und der Rezirkulation hergestellt werden kann.

Im Folgenden soll eine beispielhafte Ausführungsform erläutert werden:

> Die Verwendung des Teilintegrals als die "Größenvariable", die proportional zur Rezirkulation ist, zeigt in vitro die geringste Streuung der Messergebnisse. In Fig. 9 wäre dieses Teilintegral exemplarisch:

$$"Größen\,\mathrm{var}\,iable..G" = \sum_{50s}^{150s} Detektor_t$$

### 3.3 Die mathematische Bestimmung der Rezirkulation mittels einer Transientenmethode nach Blutflussänderung über die Zeitkonstante T

Ebenso wie die Dämpfung $\delta$ oder das Integral kann auch eine Zeitkonstante T als Parameter/"Größenvariable" zur Auswertung der zeitlichen Änderungen des physikalisch-chemischen Parameters $P_D$ herangezogen werden:

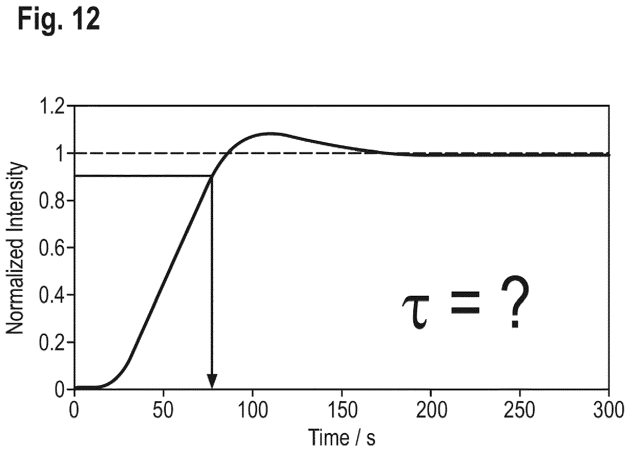

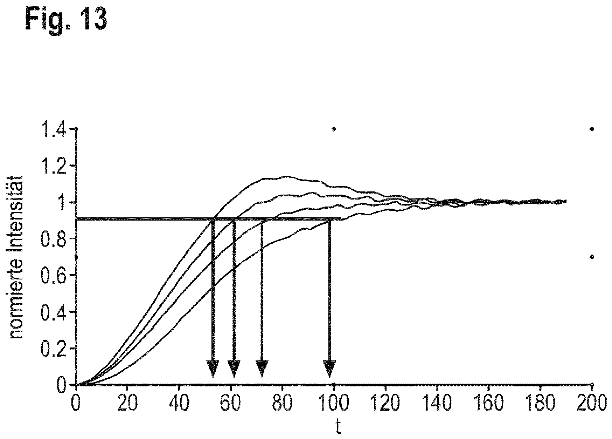

> Die Zeitkonstante T sei eine charakteristische Zeit des Signalanstiegs im UV-Detektor 6 nach einer Blutflussänderung. Exemplarisch wird diejenige Zeit T verwendet, nach der ein definierter Wert $I_x$ des normierten Intensitätssignals [f(t)] überschritten wird. Im Beispielsfalle der Figuren 12 und 13 ist $I_x$ = 0,9.

Es gilt:

$$\tau = \min(t) \mid f(t) > I_x$$

Fig. 13 zeigt die normierten Intensitätssignale bei unterschiedlichen Rezirkulationsniveaus. Je höher die Rezirkulation, desto kleiner wird die Zeitkonstante T - sie ist also dem Rezirkulationsgrad invers proportional.

Ebenso wie die Dämpfung $\delta$ und das bestimmte Integral über die Zeit, ist auch die Zeitkonstante T abhängig vom verwendeten Dialysator sowie vom verwendeten zweiten Blutfluss.

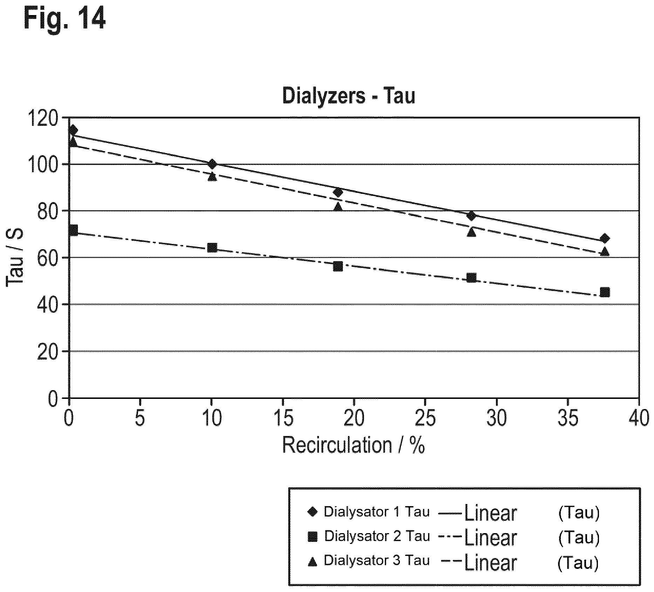

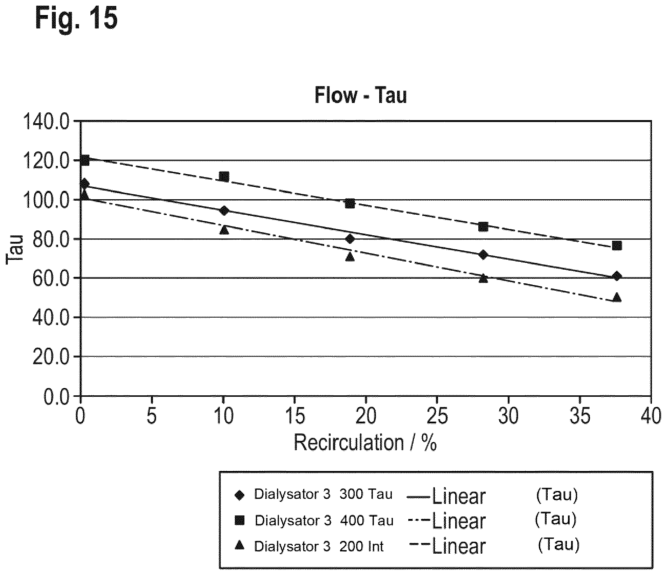

Auch bei dieser Ausführungsform der Erfindung kann eine erhöhte Messgenauigkeit durch Kenntnis der UV-Absorption als physikalisch-chemischer Parameter $P_D$ bei einem zweiten Blutfluss von 50 ml/min erzielt werden, wenn mit Hilfe eines empirisch ermittelten Faktors gemessene Wert für die Rezirkulation korrigiert wird. Die Figuren 14 und 15 stellen den Einfluss von Blutfluss und Dialysator auf die Zeitkonstante T dar.

Für jeden Dialysator existiert eine eigene Funktion, die die Zeitkonstante T mit der Rezirkulation R verknüpft und im Beispielsfalle als Polynom ersten Grades dargestellt ist. Eine Änderung des Ziel - Blutflusses bewirkt im Falle "Größenvariable G" = T eine Veränderung des Offset so wie es in Fig. 15 qualitativ dargestellt ist.

### 4. Dialysatorerkennung und Kennfelder

Generell gilt für sämtliche aufgezeigten Verfahren der Transientenanalyse "Größenvariable G" = f(Dialysator, A, BF). Diese drei Größen sind bekannt, so dass mit ihrer Hilfe aus der dialysatseitigen Messung nach Flussänderung die Rezirkulation R bestimmt werden kann.

### 4.1 Dialysatorerkennung

Bei sämtlichen beschriebenen Ausführungsformen der vorliegenden Erfindung kann die Dialysatorerkennung wie folgt erfolgen:

- Eingabe des Dialysatortyps bei Messung durch das Pflegepersonal
- Erkennung mittels RFID am Disposeable
- Erkennung mittels Barcode oder DataMax Reader am Disposeable

### 4.2 Kennfelder

Im Rahmen der praktischen Durchführung der vorliegenden Erfindung ist es vorteilhaft, eine mehrdimensionale Funktion f(A,BF) für jeden zum Einsatz kommenden Dialysator zu hinterlegen, so dass unter Zuhilfenahme der bekannten Kennfelder nicht nur eine qualitative Aussage möglich ist, d.h. eine Messung der Art:

> REZIRKULATION? →JA/NEIN, sondern - wie oben erläutert - eine quantitative Bestimmung der Rezirkulation im Shunt erfolgen kann.

Dabei lassen sich sämtliche genannten Methoden (Dämpfung $\delta$, Integralbildung und Zeitkonstante T) kombinieren, um durch Mittelung der erhaltenen Ergebnisse für die Rezirkulation R die Messgenauigkeit zu erhöhen oder wenigstens eine Plausibilitätsprüfung der Ergebnisse vorzunehmen. Diese Vorgehensweise ist möglich, da die Methoden voneinander unabhängige Ergebnisse liefern. Selbstverständlich ist dem Fachmann wohlbekannt, dass auch eine geeignet gewichtete Mittelung in Frage kommt.

Dem Fachmann ist ebenfalls bekannt, dass neben den beispielhaft beschriebenen Transientenanalyseverfahren über Dämpfung $\delta$ Integralbildung und Zeitkonstante T auch weitere hier nicht beschriebene mathematische Methoden in Betracht kommen, um aus der zeitlichen Änderung des am Sensor 6 gemessenen Parameters $P_D$ nach Blutflussänderung die Rezirkulation im Shunt zu berechnen.

5. Abwandlungen

Ob eine Rezirkulation im Shuntgefäß vorhanden ist, lässt sich grundsätzlich auch durch eine kleine Änderung des Blutflusses BF (in der nachfolgenden Gleichung mit $Q_b$ bezeichnet) ermitteln. Hierzu gilt die folgende Beziehung:

$$C_e = \left( \frac{1-R}{1-R \cdot \left(1 - \frac{C_d}{Q_b}\right)} \right) \cdot C_d$$

mit

$C_e$ als der effektiven Clearance aus Patientensicht,
R als Rezirkulation und
$C_d$ als Clearance des Dialysators.

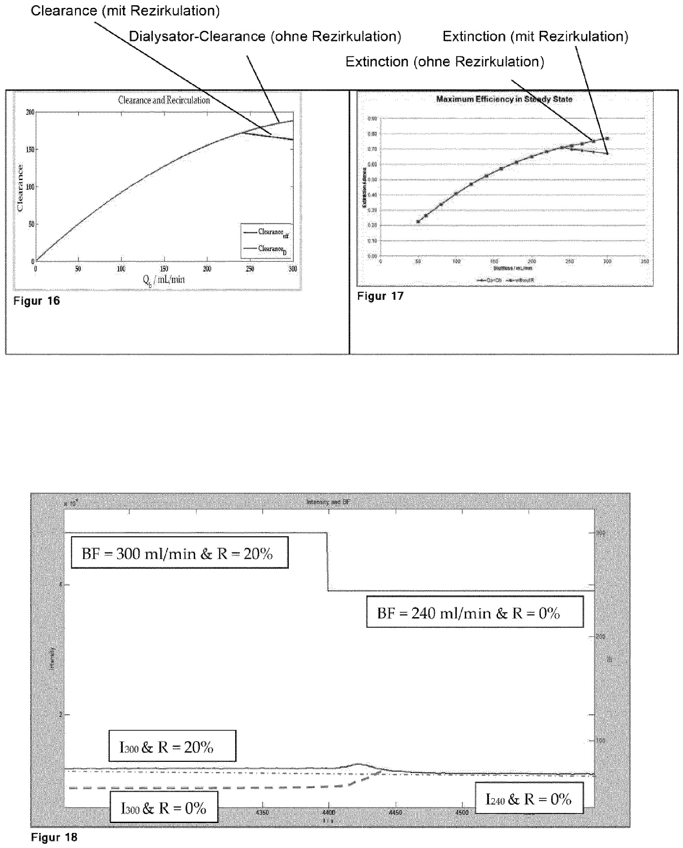

Das bedeutet, dass die Clearance durch die Rezirkulationseffekte am Patientenshunt deutlich hinter der reinen Dialysatorclearance zurücksteht, die im rezirkulationsfreien Fall vorliegen würde. Dieser Umstand wird durch die Fig. 16 illustriert. Hier ist die reine Clearance Kennlinie $K_1$ eines Dialysators A abgebildet. Diese gilt für den rezirkulationsfreien Fall und stellt sich für den rezirkulationsfreien Fall auch so aus Patientensicht dar. Als $K_2$ ist die effektive Clearance dargestellt, wenn diese aus Patientensicht durch Rezirkulationseffekte verändert (herabgesetzt) wurde. In Fig. 16 gilt beispielhaft für $Q_B$ = 300 ml/min, dass R = 20% beträgt.

Fig. 17 zeigt die mittels eines UV-Sensors gemessene Extinktion (Absorbanz) im Abfluss der Dialysemaschine. Es ist erkennbar, dass die Extinktion ein direktes Maß für die effektive Clearance ist. In Fig. 17 ist für den rezirkulationsfreien Fall wie auch für den Rezirkulationsfall die Extinktion in Abhängigkeit vom Blutfluss aufgetragen. Auch hier wurde beispielhaft von der Vorraussetzung ausgegangen, dass R (Rezirkulation) bei einem Blutfluss von 300ml/min einen Wert von 20% annimmt.

Will man nun bei einem Blutfluss $Q_{b1}$ herausfinden, ob Rezirkulation vorherrscht, dann kann dies durch Indizieren einer kleinen Blutflussänderung geschehen (z.B. $Q_{b1}$ +/- 40 ml/min). Dann wird die Veränderung der Extinktion / Intensität am UV-Sensor überwacht. Die Fig. 18 soll an einem Beispiel verdeutlichen, wie dieses Prinzip funktioniert. Es wird im vorliegenden Beispiel gemäß Fig. 18 von der folgenden Annahme ausgegangen:

I. $Q_b$ = 300 ml/min für R = 0% oder R = 20%
II. $Q_{shunt}$ = 240 ml/min für R = 20%
III. Qshunt = 300 ml/min für R = 0%

In Abhängigkeit vom Ausgangszustand ändert sich die Intensität in einer bestimmten Art und Weise, je nachdem, ob R > 0% ist oder nicht. Dadurch kann eine Überwachung des Anstiegs nach einer Blutflussänderung die Aussage liefern, ob Rezirkulation vorgelegen hat oder nicht. Die Signalanalyse erfolgt durch dem Fachmann bekannte Verfahren (z.B. HillClimber, Suche nach Vorzeichenwechsel, im Signal, Verhältnis der stationären Level, etc.).

Beschrieben wird folglich ein Steuerungsverfahren einer Dialysemaschine sowie eine Dialysemaschine zur Ermöglichung der dialysatseitigen Erfassung einer Rezirkulation in einem arteriovenösen Shunt eines Patienten während einer laufenden Hämodialyse, wobei die Dialysemaschine wenigstens einen Dialysierflüssigkeitseinlass (1) sowie wenigstens einen Dialysierflüssigkeitsabfluss (2) für eine ausgewählte Dialysierflüssigkeit und wenigstens eine Dialysierflüssigkeitspumpe (3) hat, welche in fluidischer Verbindung mit wenigstens einem Dialysator (4) stehen, der eine semipermeable Membran aufweist, welche die Grenze zwischen der Dialysierflüssigkeits-Seite und einer Blut-Seite der Dialysemaschine bildet, wobei die Dialysierflüssigkeit auf der Dialysierflüssigkeits-Seite der Membran eine Dialysierflüssigkeitskammer (4a) des Dialysators (4) in einer vorgegebenen Richtung durchströmt und wobei auf der Blut-Seite der Dialysemaschine Blut in einem extrakorporalen Kreislaufzweig mittels einer Blutpumpe (5) in Gegenrichtung zur Strömungsrichtung der Dialysierflüssigkeit durch eine Blutkammer (4b) des Dialysators (4) geführt wird, um urämische Toxine durch Diffusion über die semipermeable Membran aus dem extrakorporal geführten Blut zu entfernen,
wobei ein Sensor (6) in Dialysierflüssigkeits-Strömungsrichtung gesehen dem Dialysator (4) nachgelagert ist, der eine Änderung eines physikalisch-chemischen Parameters $P_D$ der abfließenden Dialysierflüssigkeit erfasst; und Steuerelemente (8, 9) vorgesehen sind, um in ausgewählter Weise die Dialysierflüssigkeit durch den Dialysator (4) zu führen oder daran vorbeizuleiten, mit den folgenden Verfahrensschritten (bzw. Dialysemaschinenfunktionen):

- (Mittel für) optionales Vorbeileiten der Dialysierflüssigkeit am Dialysator (4), so dass die Dialysierflüssigkeit unverändert am Sensor (6) entlang geführt wird, wodurch der Sensor (6) an der reinen Dialysierflüssigkeit kalibriert wird;

- (Mittel für) Schalten der die Steuerelemente (8, 9) nach der optionalen Sensorkalibrierung, derart dass die Dialysierflüssigkeit die Dialysatorkammer (4a) durchströmt, und Einstellen eines gewünschten ersten Blutflusswerts $BF_1$ im blutseitigen extrakorporalen Kreislaufzweig, derart, dass sich zwischen einem Eingang (20) des extrakorporalen Kreislaufzweiges und einem Ausgang (21) des extrakorporalen Kreislaufzweiges eine Rezirkulation R und über die semipermeable Membran des Dialysators (4) eine erste Clearance $K_1$ einstellen und der vom Sensor (6) erfasste Parameter einen entsprechenden Wert $P_{D1}$ annimmt;

- (Mittel für) Ändern des ersten Blutflusswerts $BF_1$ auf einen gewünschten zweiten Blutflusswert $BF_2$, wobei sich über die semipermeable Membran des Dialysators (4) eine zweite Clearance $K_2$ einstellt; und am Sensor (6) ein neuer Parameter-Wert $P_{D2}$ sowie der Änderungsverlauf von $P_{D1}$ zu $P_{D2}$ entsprechend vorliegt und erfasst wird; und

- (Mittel für) Bestimmen der Rezirkulation R anhand des Änderungsverlaufs (und/oder der Änderung) vom Parameterwert $P_{D1}$ zum Parameter-Wert $P_{D2}$.

Bezugszeichenliste

| | |
|---|---|
| 1 | Dialysierflüssigkeitseinlass |
| 2 | Dialysierflüssigkeitsabfluss |
| 3 | Dialysierflüssigkeitspumpe |
| 4 | Dialysator |
| 4a | Dialysierflüssigkeitskammer |
| 4b | Blutkammer |
| 5 | Blutpumpe |
| 6 | Sensor |
| 7 | extrakorporaler Kreislaufzweig |
| 8 | Steuereinrichtung, Ventil |
| 9 | Steuereinrichtung, Ventil |
| 20 | Eingang des extrakorporalen Kreislaufzweiges |
| 21 | Ausgang des extrakorporalen Kreislaufzweiges |
| $BF_1$ | erster Blutflusswert |
| $BF_2$ | zweiter Blutflusswert |
| $K_1$ | (erste) Clearance |
| $K_2$ | (zweite) Clearance |
| $P_D$ | physikalisch-chemischer Parameter |
| R | Rezirkulation |
| Q | Fluss |
| $\delta$ | Dämpfung |
| T | Zeitkonstante Tau |

**Patentansprüche**

1. Steuerungsverfahren einer Dialysemaschine zur Ermöglichung der dialysierflüssigkeitsseitigen Erfassung einer Rezirkulation in einem arteriovenösen Ein-/Auslass-Element, vorzugsweise Shunt, wobei die Dialysemaschine wenigstens einen Dialysierflüssigkeitseinlass (1) sowie wenigstens einen Dialysierflüssigkeitsabfluss (2) für eine ausgewählte Dialysierflüssigkeit und wenigstens eine Dialysierflüssigkeitspumpe (3) hat, welche in fluidischer Verbindung mit wenigstens einem Dialysator (4) stehen, wobei ein Sensor (6) in Dialysierflüssigkeits-Strömungsrichtung gesehen dem Dialysator (4) nachgelagert ist, der eine Änderung eines physikalisch- chemischen Parameters ($P_D$) der abfließenden Dialysierflüssigkeit erfasst,
mit den folgenden Verfahrensschritten:

- Einstellen eines gewünschten ersten Blutflusswerts $BF_1$, bei dem sich eine Rezirkulation R $>/= 0$ einstellt und der vom Sensor (6) erfasste Parameter einen entsprechenden Wert $P_{D1}$ annimmt;
- Ändern des ersten Blutflusswerts BF1 auf einen vorzugsweise kleineren zweiten Blutflusswert $BF_2$, wobei am Sensor (6) ein neuer Parameter-Wert $P_{D2}$ entsprechend vorliegt und erfasst wird;
- bolusloses Bestimmen der Rezirkulation R anhand des Änderungsverlaufs und/oder der Änderung vom Parameter-Wert $P_{D1}$ zum Parameter-Wert $P_{D2}$, und
- Erfassen der Rezirkulation R durch Auswerten eines Einschwingverhaltens des zeitlichen Verlaufs der Änderung des physikalisch-chemischen Parameters $P_D$ vom Parameter-Wert $P_{D1}$ zum Parameter-Wert $P_{D2}$ nach Änderung des ersten Blutflusses $BF_1$ auf den zweiten Blutfluss $BF_2$ oder umgekehrt,

**dadurch gekennzeichnet, dass**
das Einschwingverhalten durch eine Dämpfung $\delta$ erfasst wird und mittels der Dämpfung $\delta$ des Einschwingverhaltens nach Anlegen des zweiten Blutflusses $BF_2$ die Rezirkulation R bestimmt wird.

2. Steuerungsverfahren einer Dialysemaschine zur Ermöglichung der dialysierflüssigkeitsseitigen Erfassung einer Rezirkulation in einem arteriovenösen Ein-/Auslass-Element, vorzugsweise Shunt, wobei die Dialysemaschine wenigstens einen Dialysierflüssigkeitseinlass (1) sowie wenigstens einen Dialysierflüssigkeitsabfluss (2) für eine ausgewählte Dialysierflüssigkeit und wenigstens eine Dialysierflüssigkeitspumpe (3) hat, welche in fluidischer Verbindung mit wenigstens einem Dialysator (4) stehen, wobei ein Sensor (6) in Dialysierflüssigkeits-Strömungsrichtung gesehen dem Dialysator (4) nachgelagert ist, der eine Änderung eines physikalisch- chemischen Parameters ($P_D$) der abfließenden Dialysierflüssigkeit erfasst,

mit den folgenden Verfahrensschritten:

- Einstellen eines gewünschten ersten Blutflusswerts $BF_1$, bei dem sich eine Rezirkulation R >/= 0 einstellt und der vom Sensor (6) erfasste Parameter einen entsprechenden Wert $P_{D1}$ annimmt;
- Ändern des ersten Blutflusswerts $BF1$ auf einen vorzugsweise kleineren zweiten Blutflusswert $BF_2$, wobei am Sensor (6) ein neuer Parameter-Wert $P_{D2}$ entsprechend vorliegt und erfasst wird;
- bolusloses Bestimmen der Rezirkulation R anhand des Änderungsverlaufs und/oder der Änderung vom Parameter-Wert $P_{D1}$ zum Parameter-Wert $P_{D2}$, und
- Erfassen der Rezirkulation R durch Auswerten eines Einschwingverhaltens des zeitlichen Verlaufs der Änderung des physikalisch-chemischen Parameters $P_D$ vom Parameter-Wert $P_{D1}$ zum Parameter-Wert $P_{D2}$ nach Änderung des ersten Blutflusses $BF_1$ auf den zweiten Blutfluss $BF_2$ oder umgekehrt,

**dadurch gekennzeichnet, dass**

das Einschwingverhalten nach Anlegen des zweiten Blutflusses BF2 durch eine charakteristische Zeit T eines Signalanstiegs des vom Sensor (6) gelieferten Ausgangssignals erfasst wird und hiermit die Rezirkulation R bestimmt wird.

3. Verfahren nach Anspruch 1 oder 2, **dadurch gekennzeichnet, dass** als physikalisch-chemischer Parameter $P_D$ eine optische Absorption, insbesondere eine Absorption im IR-Wellenlängenbereich, UV-Wellenlängenbereich oder im sichtbaren Wellenlängenbereich oder eine Absorbanz, verwendet wird.

4. Verfahren nach Anspruch 3, **dadurch gekennzeichnet, dass** als physikalisch-chemischer Parameter PD eine optische Absorption im UV-Wellenlängenbereich, insbesondere bei einer Wellenlänge von ca. 280 nm oder eine Absorbanz verwendet wird, und dass als Sensor (6) ein UV-Detektor verwendet wird.

5. Verfahren nach einem der vorhergehenden Ansprüche, **dadurch gekennzeichnet, dass** der zweite Blutflusswert $BF_2$ kleiner ist als der erste Blutflusswert $BF_1$, wobei insbesondere gilt: $BF_2 = r \times BF_1$, mit r = 0,05 bis 0,595; oder dass der zweite Blutflusswert $BF_2$ größer ist als der erste Blutflusswert $BF_1$ wobei insbesondere gilt: $BF_2 = r \times BF_1$, mit r = 1,05 bis 20.

6. Verfahren nach Anspruch 1 oder 2, **dadurch gekennzeichnet, dass** das Einschwingverhalten nach Anlegen des zweiten Blutflusses $BF_2$ durch eine Integration eines normierten Zeitsignals, insbesondere einer vom Sensor (6) ausgegebenen Intensität, über einen bestimmten Zeitbereich, erfasst wird und hiermit die Rezirkulation R bestimmt wird.

7. Dialysemaschine mit einer Einrichtung zur Erfassung einer Rezirkulation in einem arteriovenösen Ein-/Auslass-Element, vorzugsweise Shunt, wobei

die Dialysemaschine eine Dialysierflüssigkeits-Seite und eine Blut-Seite aufweist; wobei auf der Dialysierflüssigkeits-Seite wenigstens ein Dialysierflüssigkeitseinlass (1) sowie wenigstens ein Dialysierflüssigkeitsabfluss (2) für eine ausgewählte Dialysierflüssigkeit vorgesehen sind, welche in fluidischer Verbindung mit wenigstens einem Dialysator (4) stehen und mit wenigstens einem Sensor (6) zum Erfassen und vorzugsweise Speichern eines Änderungsverlaufs und/oder einer Änderung eines physikalisch-chemischen Parameters $P_D$ der aus dem Dialysator (4) abfließenden Dialysierflüssigkeit;
die Dialysemaschine dafür angepasst ist, dass im blutseitigen extrakorporalen Kreislaufzweig ein gewünschter erster Blutflusswert $BF_1$ einstellbar ist, wodurch sich zwischen einem Eingang (20) des extrakorporalen Kreislaufzweiges und einem Ausgang (21) des extrakorporalen Kreislaufzweiges eine Rezirkulation R >/= 0 einstellt und der vom Sensor (6) erfasste Parameter einen dementsprechenden Wert $P_{D1}$ annimmt;
der erste Blutflusswert $BF_1$ auf einen zweiten Blutflusswert $BF_2$ änderbar ist und somit am Sensor (6) ein dementsprechender neuer Parameter- Wert $P_{D2}$ vorliegt;
Mittel vorgesehen sind, die daran angepasst sind, boluslos den Änderungsverlauf und/oder die Änderung vom Parameter-Wert $P_{D1}$ zum Parameter-Wert $P_{D2}$ zu erfassen und daraus die Rezirkulation R vorzugsweise nach einem Verfahren gemäß einem der Ansprüche 1 bis 6 zu bestimmen; und

- die Rezirkulation R durch Auswerten eines Einschwingverhaltens des zeitlichen Verlaufs der Änderung des physikalisch-chemischen Parameters $P_D$ nach Änderung des ersten Blutflusses $BF_1$ auf den zweiten Blutfluss $BF_2$ oder umgekehrt erfasst wird,

**dadurch gekennzeichnet, dass**

das Einschwingverhalten durch eine Dämpfung $\delta$ erfasst wird oder erfassbar ist und mittels der Dämpfung $\delta$ des Einschwingverhaltens nach

Anlegen des zweiten Blutflusses BF2 die Rezirkulation R mittels eines Prozessors berechnet wird oder berechenbar ist.

8. Dialysemaschine mit einer Einrichtung zur Erfassung einer Rezirkulation in einem arteriovenösen Ein-/Auslass-Element, vorzugsweise Shunt, wobei

die Dialysemaschine eine Dialysierflüssigkeits-Seite und eine Blut-Seite aufweist; wobei auf der Dialysierflüssigkeits-Seite wenigstens ein Dialysierflüssigkeitseinlass (1) sowie wenigstens ein Dialysierflüssigkeitsabfluss (2) für eine ausgewählte Dialysierflüssigkeit vorgesehen sind, welche in fluidischer Verbindung mit wenigstens einem Dialysator (4) stehen und mit wenigstens einem Sensor (6) zum Erfassen und vorzugsweise Speichern eines Änderungsverlaufs und/oder einer Änderung eines physikalisch-chemischen Parameters $P_D$ der aus dem Dialysator (4) abfließenden Dialysierflüssigkeit; die Dialysemaschine dafür angepasst ist, dass im blutseitigen extrakorporalen Kreislaufzweig ein gewünschter erster Blutflusswert $BF_1$ einstellbar ist, wodurch sich zwischen einem Eingang (20) des extrakorporalen Kreislaufzweiges und einem Ausgang (21) des extrakorporalen Kreislaufzweiges eine Rezirkulation R >/= 0 einstellt und der vom Sensor (6) erfasste Parameter einen dementsprechenden Wert $P_{D1}$ annimmt; der erste Blutflusswert $BF_1$ auf einen zweiten Blutflusswert $BF_2$ änderbar ist und somit am Sensor (6) ein dementsprechender neuer Parameter- Wert $P_{D2}$ vorliegt; Mittel vorgesehen sind, die daran angepasst sind, boluslos den Änderungsverlauf und/oder die Änderung vom Parameter-Wert $P_{D1}$ zum Parameter-Wert $P_{D2}$ zu erfassen und daraus die Rezirkulation R vorzugsweise nach einem Verfahren gemäß einem der Ansprüche 1 bis 6 zu bestimmen; und

- die Rezirkulation R durch Auswerten eines Einschwingverhaltens des zeitlichen Verlaufs der Änderung des physikalisch-chemischen Parameters $P_D$ nach Änderung des ersten Blutflusses $BF_1$ auf den zweiten Blutfluss $BF_2$ oder umgekehrt erfasst wird,

**dadurch gekennzeichnet, dass**

das Einschwingverhalten nach Anlegen des zweiten Blutflusses BF2 durch eine charakteristische Zeit T eines Signalanstiegs des vom Sensor (6) gelieferten Ausgangssignals erfasst wird oder erfassbar ist und hiermit die Rezirkulation R mittels eines Prozessors berechnet wird oder ermittelbar ist.

9. Dialysemaschine nach Anspruch 7 oder 8, **dadurch gekennzeichnet, dass** der physikalisch-chemischer Parameter $P_D$ eine optische Absorption, insbesondere eine Absorption im IR-Wellenlängenbereich, UV-Wellenlängenbereich oder im sichtbaren Wellenlängenbereich oder eine Absorbanz, ist.

10. Dialysemaschine nach Anspruch 9, **dadurch gekennzeichnet, dass** der physikalisch-chemischer Parameter $P_D$ eine optische Absorption im UV-Wellenlängenbereich, insbesondere bei einer Wellenlänge von ca. 280 nm oder eine Absorbanz ist, und dass der Sensor (6) ein UV-Detektor ist.

11. Dialysemaschine nach einem der Ansprüche 7 bis 10, **dadurch gekennzeichnet, dass** der zweite Blutflusswert BF2 kleiner ist als der erste Blutflusswert BF1, wobei vorzugsweise gilt: BF2 = r x BF1, mit r = 0,05 bis 0,95; oder dass der zweite Blutflusswert BF2 größer ist als der erste Blutflusswert BF1 wobei vorzugsweise gilt: BF2 = r x BF1, mit r = 1,05 bis 20.

12. Dialysemaschine nach Anspruch 7 oder 8, **dadurch gekennzeichnet, dass** das Einschwingverhalten nach Anlegen des zweiten Blutflusses BF2 durch eine Integration eines normierten Zeitsignals, vorzugsweise einer vom Sensor (6) ausgegebenen Intensität, über einen bestimmten Zeitbereich, erfasst wird oder erfassbar ist und hiermit die Rezirkulation R mittels eines Prozessors berechnet wird oder berechenbar ist.

13. Dialysemaschine nach einem der vorhergehenden Ansprüche 7 bis 12, **dadurch gekennzeichnet, dass** Drucksensoren im proximalen Kreislauf für den arteriellen Druck (PA), den venösen Druck (PV) und den Eingangsdruck (PBE) für den Dialysator (4) vorgesehen sind.

**Claims**

1. A control method of a dialysis machine to allow detection on the dialysis fluid side of recirculation in an arteriovenous inlet/outlet element, preferably a shunt, wherein the dialysis machine comprises at least one dialysis fluid inlet (1) and at least one dialysis fluid discharge (2) for a selected dialysis fluid and at least one dialysis fluid pump (3) which are in fluidic communication with at least one dialyser (4), wherein, viewed in the dialysis fluid flow direction, a sensor (6) is arranged downstream of the dialyser (4) and detects a change of a physicochemical parameter ($P_D$) of the outflowing dialysis fluid, comprising the following method steps of:

- setting a desired first blood flow value $BF_1$ at

which recirculation R >/= 0 is adjusted and the parameter detected by the sensor (6) adopts an appropriate value $P_{D1}$;

- changing the first blood flow value $BF_1$ to a preferably lower second blood flow value $BF_2$, wherein at the sensor (6) a new parameter value $P_{D2}$ is appropriately provided and detected;

- determining without bolus the recirculation R by way of the course of change and/or the change from the parameter value $P_{D1}$ to the parameter value $P_{D2}$, and

- detecting the recirculation R by evaluating a transient behavior of the time course of the change of the physicochemical parameter $P_D$ from the parameter value $P_{D1}$ to the parameter value $P_{D2}$ after change of the first blood flow $BF_1$ to the second blood flow $BF_2$ or vice versa,

**characterized in that**

the transient behavior is detected by a damping $\delta$ and the recirculation R is determined by means of the damping $\delta$ of the transient behavior after applying the second blood flow $BF_2$.

2. A control method of a dialysis machine to allow detection on the dialysis fluid side of recirculation in an arteriovenous inlet/outlet element, preferably a shunt, wherein the dialysis machine comprises at least one dialysis fluid inlet (1) and at least one dialysis fluid discharge (2) for a selected dialysis fluid and at least one dialysis fluid pump (3) which are in fluidic communication with at least one dialyser (4), wherein, viewed in the dialysis fluid flow direction, a sensor (6) is arranged downstream of the dialyser (4) and detects a change of a physicochemical parameter ($P_D$) of the outflowing dialysis fluid, comprising the following method steps of:

- setting a desired first blood flow value $BF_1$ at which recirculation R >/= 0 is adjusted and the parameter detected by the sensor (6) adopts an appropriate value $P_{D1}$;

- changing the first blood flow value $BF_1$ to a preferably lower second blood flow value $BF_2$, wherein at the sensor (6) a new parameter value $P_{D2}$ is appropriately provided and detected;

- determining without bolus the recirculation R by way of the course of change and/or the change from the parameter value $P_{D1}$ to the parameter value $P_{D2}$, and

- detecting the recirculation R by evaluating a transient behavior of the time course of the change of the physicochemical parameter $P_D$ from the parameter value $P_{D1}$ to the parameter value $P_{D2}$ after change of the first blood flow $BF_1$ to the second blood flow $BF_2$ or vice versa,

**characterized in that**

the transient behavior is detected after applying the second blood flow $BF_2$ by a characteristic time $\top$ of a signal increase of the output signal supplied by the sensor (6) and hereby the recirculation R is determined.

3. The method according to claim 1 or 2, **characterized in that** as physicochemical parameter $P_D$ an optical absorption, especially an absorption in the IR wavelength range, UV wavelength range or in the visible wavelength range, or an absorbance is utilized.

4. The method according to claim 3, **characterized in that** as physicochemical parameter $P_D$ an optical absorption in the UV wavelength range, especially with a wavelength of approx. 280 nm, or an absorbance is utilized and that a UV detector is used as sensor (6).

5. The method according to any one of the preceding claims, **characterized in that** the second blood flow value $BF_2$ is lower than the first blood flow value $BF_1$, wherein it is especially applicable: $BF_2 = r \times BF_1$, wherein r = 0.05 to 0.595; or that the second blood flow value $BF_2$ is higher than the first blood flow value $BF_1$, wherein especially $BF_2 = r \times BF_1$ is applicable, with r = 1.05 to 20.

6. The method according to claim 1 or 2, **characterized in that** the transient behavior is detected after applying the second blood flow $BF_2$ by integrating a normalized time signal, especially having an intensity output by the sensor (6), over a particular time range and hereby the recirculation R is determined.

7. A dialysis machine comprising means for detecting recirculation in an arteriovenous inlet/outlet element, preferably a shunt, wherein

the dialysis machine has a dialysis fluid side and a blood side; wherein on the dialysis fluid side at least one dialysis fluid inlet (1) as well as at least one dialysis fluid discharge (2) for a selected dialysis fluid is provided which are in fluidic communication with at least one dialyser (4), and comprising at least one sensor (6) for detecting and preferably storing a course of change and/or a change of a physicochemical parameter $P_D$ of the dialysis fluid outflowing from the dialyser (4);

the dialysis machine is adapted to a desired first blood flow value $BF_1$ being adjustable in the blood-side extracorporeal circulatory branch, whereby between an inlet (20) of the extracorporeal circulatory branch and an outlet (21) of the extracorporeal circulatory branch a recirculation R >/= 0 is adjusted and the parameter detected by the sensor (6) adopts an appropriate value $P_{D1}$;

the first blood flow value $BF_1$ is adapted to be changed to a second blood flow value $BF_2$ and thus at the sensor (6) a corresponding new parameter value $P_{D2}$ is provided;

means are provided which are adapted to detect without bolus the course of change and/or the change from the parameter value $P_{D1}$ to the parameter value $P_{D2}$ and to determine therefrom the recirculation R preferably according to a method according to any one of the claims 1 to 6; and

the recirculation R is detected by evaluating a transient behavior of the time course of the change of the physicochemical parameter $P_D$ after change of the first blood flow $BF_1$ to the second blood flow $BF_2$ or vice versa,

**characterized in that**

the transient behavior is detected or detectable by a damping $\delta$ and by means of the damping $\delta$ of the transient behavior after applying the second blood flow $BF_2$ the recirculation R is calculated or can be calculated by means of a processor.

8. A dialysis machine comprising means for detecting recirculation in an arteriovenous inlet/outlet element, preferably a shunt, wherein

the dialysis machine has a dialysis fluid side and a blood side; wherein on the dialysis fluid side at least one dialysis fluid inlet (1) as well as at least one dialysis fluid discharge (2) for a selected dialysis fluid is provided which are in fluidic communication with at least one dialyser (4), and comprising at least one sensor (6) for detecting and preferably storing a course of change and/or a change of a physicochemical parameter $P_D$ of the dialysis fluid outflowing from the dialyser (4);

the dialysis machine is adapted to a desired first blood flow value $BF_1$ being adjustable in the blood-side extracorporeal circulatory branch, whereby between an inlet (20) of the extracorporeal circulatory branch and an outlet (21) of the extracorporeal circulatory branch a recirculation R >/= 0 is adjusted and the parameter detected by the sensor (6) adopts an appropriate value $P_{D1}$;

the first blood flow value $BF_1$ is adapted to be changed to a second blood flow value $BF_2$ and thus at the sensor (6) a corresponding new parameter value $P_{D2}$ is provided;

means are provided which are adapted to detect without bolus the course of change and/or the change from the parameter value $P_{D1}$ to the parameter value $P_{D2}$ and to determine therefrom the recirculation R preferably according to a method according to any one of the claims 1 to 6; and

the recirculation R is detected by evaluating a transient behavior of the time course of the change of the physicochemical parameter $P_D$ after change of the first blood flow $BF_1$ to the second blood flow $BF_2$ or vice versa,

**characterized in that**

the transient behavior is detected or detectable after applying the second blood flow $BF_2$ by a characteristic time T of a signal increase of the output signal supplied by the sensor (6) and hereby the recirculation R is calculated or can be established by means of a processor.

9. The dialysis machine according to claim 7 or 8, **characterized in that** the physicochemical parameter $P_D$ is an optical absorption, especially an absorption within the IR wavelength range, UV wavelength range or in the visible wavelength range, or an absorbance.

10. The dialysis machine according to claim 9, **characterized in that** the physicochemical parameter $P_D$ is an optical absorption within the UV wavelength range, especially at a wavelength of approx. 280 nm, or an absorbance, and that the sensor (6) is a UV detector.

11. The dialysis machine according to any one of the claims 7 to 10, **characterized in that** the second blood flow value $BF_2$ is lower than the first blood flow value $BF_1$, wherein preferably $BF_2 = r \times BF_1$ is applicable, with r = 0.05 to 0.95; or that the second blood flow value $BF_2$ is higher than the first blood flow value $BF_1$, wherein preferably $BF_2 = r \times BF_1$ is applicable, with r = 1.05 to 20.

12. The dialysis machine according to claim 7 or 8, **characterized in that** the transient behavior is detected or detectable after applying the second blood flow $BF_2$ by integration of a normalized time signal, preferably an intensity output by the sensor (6), over a definite time range and hereby the recirculation R is calculated or can be calculated by means of a processor.

13. The dialysis machine according to any one of the preceding claims 7 to 12, **characterized in that** pressure sensors are provided in the proximal circulation for the arterial pressure (PA), the venous pressure (PV) and the entrance pressure (PBE) for the dialyser (4).

**Revendications**

1. Procédé de commande d'une machine de dialyse permettant la mesure, du côté du liquide de dialyse, d'une remise en circulation dans un élément d'entrée/sortie artério-veineux, de préférence un shunt, la machine de dialyse comprenant au moins une entrée de liquide de dialyse (1) ainsi qu'au moins une évacuation de liquide de dialyse (2) pour un liquide de dialyse sélectionné et au moins une pompe de liquide de dialyse (3), qui sont en liaison fluidique avec au moins un dialyseur (4), un capteur (6) étant situé dans la direction d'écoulement du liquide de dialyse, en aval du dialyseur (4), qui mesure une variation d'un paramètre physico-chimique ($P_D$) du liquide de dialyse sortant, avec les étapes suivantes :

   - réglage d'une première valeur de débit sanguin souhaitée $BF_1$, pour laquelle une remise en circulation R >/= 0 s'ajuste et le paramètre mesuré par le capteur (6) prend une valeur $P_{D1}$ correspondante ;
   - modification de la première valeur de débit sanguin $BF_1$ à une deuxième valeur de débit sanguin $BF_2$, de préférence inférieure, une nouvelle valeur de paramètre $P_{D2}$ existant de manière correspondante et étant mesurée au niveau du capteur (6) ;
   - détermination sans bolus de la remise en circulation R à l'aide du profil de variation et/ou de la variation de la valeur de paramètre $P_{D1}$ à la valeur de paramètre $P_{D2}$ et
   - mesure de la remise en circulation R par analyse d'un comportement oscillatoire du tracé en fonction du temps de la variation du paramètre physico-chimique $P_D$ de la valeur de paramètre $P_{D1}$ à la valeur de paramètre $P_{D2}$ après la variation du premier débit sanguin $BF_1$ vers le deuxième débit sanguin $BF_2$ ou inversement,

   **caractérisé en ce que**
   le comportement oscillatoire est mesuré par un amortissement δ et, au moyen de l'amortissement δ du comportement oscillatoire, après l'application du deuxième débit sanguin $BF_2$, la remise en circulation R est déterminée.

2. Procédé de commande d'une machine de dialyse permettant la mesure, du côté du liquide de dialyse, d'une remise en circulation dans un élément d'entrée/sortie artério-veineux, de préférence un shunt, la machine de dialyse comprenant au moins une entrée de liquide de dialyse (1) ainsi qu'au moins une évacuation de liquide de dialyse (2) pour un liquide de dialyse sélectionné et au moins une pompe de liquide de dialyse (3), qui sont en liaison fluidique avec au moins un dialyseur (4), un capteur (6) étant situé dans la direction d'écoulement du liquide de dialyse, en aval du dialyseur (4), qui mesure une variation d'un paramètre physico-chimique ($P_D$) du liquide de dialyse sortant, avec les étapes suivantes :

   - réglage d'une première valeur de débit sanguin souhaitée $BF_1$, pour laquelle une remise en circulation R >/= 0 s'ajuste et le paramètre mesuré par le capteur (6) prend une valeur $P_{D1}$ correspondante ;
   - modification de la première valeur de débit sanguin $BF_1$ à une deuxième valeur de débit sanguin $BF_2$, de préférence inférieure, une nouvelle valeur de paramètre $P_{D2}$ existant de manière correspondante et étant mesurée au niveau du capteur (6) ;
   - détermination sans bolus de la remise en circulation R à l'aide du profil de variation et/ou de la variation de la valeur de paramètre $P_{D1}$ à la valeur de paramètre $P_{D2}$ et
   - mesure de la remise en circulation R par analyse d'un comportement oscillatoire du tracé en fonction du temps de la variation du paramètre physico-chimique $P_D$ de la valeur de paramètre $P_{D1}$ à la valeur de paramètre $P_{D2}$ après la variation du premier débit sanguin $BF_1$ vers le deuxième débit sanguin $BF_2$ ou inversement,

   **caractérisé en ce que**
   le comportement oscillatoire est mesuré après l'application du deuxième débit sanguin $BF_2$ par un temps caractéristique τ d'une augmentation de signal du signal de sortie délivré par le capteur (6) et la remise en circulation R est ainsi déterminée.

3. Procédé selon la revendication 1 ou 2, **caractérisé en ce que**, en tant que paramètre physico-chimique $P_D$, une absorption optique, plus particulièrement une absorption dans le domaine de longueurs d'onde IR, le domaine de longueurs UV ou dans le domaine de longueurs d'onde visible, ou un coefficient d'absorption, est utilisé.

4. Procédé selon la revendication 3, **caractérisé en ce que**, en tant que paramètre physico-chimique PD, une absorption optique dans le domaine de longueurs d'onde UV, plus particulièrement pour une longueur d'onde d'environ 280 nm ou un coefficient d'absorption est utilisé, et **en ce que**, en tant que capteur (6), un détecteur UV est utilisé.

5. Procédé selon l'une des revendications précédentes, **caractérisé en ce que** la deuxième valeur de débit sanguin $BF_2$ est inférieure à la première valeur de débit sanguin $BF_1$, plus particulièrement : $BF_2$ = r x $BF_1$, avec r = 0,05 à 0,595 ; ou **en ce que** la deuxième valeur de débit sanguin $BF_2$ est supérieu-

re à la première valeur de débit sanguin $BF_1$, plus particulièrement : $BF_2 = r \times BF_1$, avec r = 1,05 à 20.

6. Procédé selon la revendication 1 ou 2, **caractérisé en ce que** le comportement oscillatoire est déterminé après l'application du deuxième débit sanguin $BF_2$ par une intégration d'un signal de temps normé, plus particulièrement d'une intensité délivrée par le capteur (6), sur une période déterminée et la remise en circulation R est ainsi déterminée.

7. Machine de dialyse avec un dispositif de mesure d'une remise en circulation dans un élément d'entrée/sortie artério-veineux, de préférence un shunt, la machine de dialyse comprenant un côté de liquide de dialyse et un côté sanguin ; moyennant quoi, sur le côté liquide de dialyse, se trouvent au moins une entrée de liquide de dialyse (1) ainsi qu'au moins une évacuation de liquide de dialyse (2) pour un liquide de dialyse sélectionné, qui sont en liaison fluidique avec au moins un dialyseur (4) et avec au moins un capteur (6) pour la mesure et de préférence l'enregistrement d'un tracé de variation et/ou d'une variation d'un paramètre physico-chimique $P_D$ du liquide de dialyse sortant du dialyseur (4) ; la machine de dialyse étant conçue de façon à ce que, dans la branche du circuit extracorporel du côté du sang, une première valeur de débit sanguin souhaitée $BF_1$ peut être réglée, ce qui permet d'ajustement d'une remise en circulation R >/= 0 entre une entrée (20) de la branche de circuit extracorporel et une sortie (21) de la branche de circuit extracorporel, et le paramètre mesuré par le capteur (6) prend une valeur $P_{D1}$ correspondante ; la première valeur de débit sanguin $BF_1$ pouvant être modifiée vers une deuxième valeur de débit sanguin $BF_2$ et une nouvelle valeur de paramètre $P_{D2}$ correspondante existe donc au niveau du capteur (6) ; des moyens étant prévus, qui sont conçus pour mesurer, sans bolus, le tracé de variation et/ou la variation de la valeur de paramètre $P_{D1}$ à la valeur de paramètre $P_{D2}$ et pour en déduire la remise en circulation R de préférence selon un procédé selon l'une des revendications 1 à 6 ; et la remise en circulation R étant mesurée par analyse d'un comportement oscillatoire du tracé en fonction du temps de la variation du paramètre physico-chimique $P_D$ après la variation du premier débit sanguin $BF_1$ vers le deuxième débit sanguin $BF_2$ ou inversement, **caractérisé en ce que** le comportement oscillatoire est mesuré ou peut être mesurée à l'aide d'un amortissement $\delta$ et, au moyen de l'amortissement $\delta$ du comportement oscillatoire, après l'application du deuxième débit sanguin BF2, la remise en circulation R est calculé ou peut être calculé au moyen d'un processeur.

8. Machine de dialyse avec un dispositif de mesure d'une remise en circulation dans un élément d'entrée/sortie artério-veineux, de préférence un shunt, la machine de dialyse comprenant un côté de liquide de dialyse et un côté sanguin ; moyennant quoi, sur le côté du liquide de dialyse, se trouvent au moins une entrée de liquide de dialyse (1) ainsi qu'au moins une évacuation de liquide de dialyse (2) pour un liquide de dialyse sélectionné, qui sont en liaison fluidique avec au moins un dialyseur (4) et avec au moins un capteur (6) pour la mesure et de préférence l'enregistrement d'un tracé de variation et/ou d'une variation d'un paramètre physico-chimique $P_D$ du liquide de dialyse sortant du dialyseur (4) ; la machine de dialyse étant conçue de façon à ce que, dans la branche du circuit extracorporel côté sang, une première valeur de débit sanguin souhaitée $BF_1$ puisse être réglée, ce qui permet d'ajuster une remise en circulation R >/= 0 entre une entrée (20) de la branche de circuit extracorporel et une sortie (21) de la branche de circuit extracorporel et le paramètre mesuré par le capteur (6) prend une valeur $P_{D1}$ correspondante ; la première valeur de débit sanguin $BF_1$ pouvant être modifiée à une deuxième valeur de débit sanguin $BF_2$ et une nouvelle valeur de paramètre $P_{D2}$ correspondante existe donc au niveau du capteur (6) ; des moyens étant prévus, qui sont conçus pour mesurer sans bolus le tracé de la variation et/ou la variation de la valeur de paramètre $P_{D1}$ à la valeur de paramètre $P_{D2}$ et d'en déduire la remise en circulation R de préférence selon un procédé selon l'une des revendications 1 à 6 ; et la remise en circulation R étant mesurée par analyse d'un comportement oscillatoire du tracé en fonction du temps de la variation de la valeur du paramètre physico-chimique $P_D$ après la variation du premier débit sanguin $BF_1$ vers le deuxième débit sanguin $BF_2$ ou inversement, **caractérisé en ce que** le comportement oscillatoire est mesuré ou peut être mesuré après l'application du deuxième débit sanguin $BF_2$ à l'aide d'un temps caractéristique $\tau$ d'une augmentation de signal du signal de sortie délivré par le capteur (6) et la remise en circulation R est calculé ou peut être déterminé au moyen d'un processeur.

9. Machine de dialyse selon la revendication 7 ou 8, **caractérisée en ce que** le paramètre physico-chimique $P_D$ est une absorption optique, plus particulièrement une absorption dans le domaine de longueurs d'ondes IR, le domaine de longueurs d'ondes UV ou dans le domaine de longueurs d'ondes visibles ou un coefficient d'absorption.

10. Machine de dialyse selon la revendication 9, **caractérisée en ce que** le paramètre physico-chimique

$P_D$ est une absorption optique dans le domaine de longueurs d'ondes UV, plus particulièrement à une longueur d'onde d'environ 280 nm ou un coefficient d'absorption, et **en ce que** le capteur (6) est un détecteur UV.

11. Machine de dialyse selon l'une des revendications 7 à 10, **caractérisée en ce que** la deuxième valeur de débit sanguin BF2 est inférieure à la première valeur de débit sanguin BF1, de préférence : BF2 = r x BF1, avec r = 0,05 à 0,95 ; ou **en ce que** la deuxième valeur de débit sanguin BF2 est supérieure à la première valeur de débit sanguin BF1, de préférence : BF2 = r x BF1, avec r = 1,05 à 20.

12. Machine de dialyse selon la revendication 7 ou 8, **caractérisée en ce que** le comportement oscillatoire est mesuré ou peut être mesuré après l'application du deuxième débit sanguin BF2 par une intégration d'un signal de temps normé, de préférence d'une intensité délivrée par le capteur (6), sur une période déterminée, et la remise en circulation R est ainsi calculée ou peut être calculée au moyen d'un processeur.

13. Machine de dialyse selon l'une des revendications 7 à 12, **caractérisée en ce que** des capteurs de pression sont prévus dans un circuit proximal pour la pression artérielle (PA), la pression veineuse (PV) et la pression d'entrée (PBE) pour le dialyseur (4).

Fig. 1

Fig. 2

Fig. 3

# Fig. 4

(Blutfluss-Änderung)

(effektive Clearance verschiebt sich bei Rezirkulation.
Daher ändert sich $Level_0$ und $Level_R$)

## Fig. 5

Korrelation zwischen delta und
der Rezirkulation im Shunt

## Fig. 6

Korrelation Rezirkulation - (1-delta)

x-axis: 1-delta, y-axis: Rezirkulation

## Fig. 7

**Dialyzers - 1-D**

Legend:
- ◆ Dialysator 1  —— Linear  (1 - D)
- ■ Dialysator 2  ---- Linear  (1 - D)
- ▲ Dialysator 3  -- Linear  (1 - D)

x-axis: Recirculation / %
y-axis: 1-D

Zeichnungsfläche

## Fig. 8

**Blutfluss - 1-D**

Legend:
- ◆ Dialysator 3  300 1-D  —Linear
- ■ Dialysator 3  400 1-D  ---Linear
- ▲ Dialysator 3  200 1-D  --Linear

Fig. 9

## Fig. 10

**Dialyzers - Intergral**

Legend:
- ◆ Dialysator 1 —— Linear
- ■ Dialysator 2 --- Linear
- ▲ Dialysator 3 −− Linear

Y-axis: Integral
X-axis: Recirculation / %

## Fig. 11

## Fig. 12

Fig. 13

Fig. 14

Dialyzers - Tau

◆ Dialysator 1 Tau ——Linear (Tau)
■ Dialysator 2 Tau ---Linear (Tau)
▲ Dialysator 3 Tau --Linear (Tau)

# Fig. 15

**Flow - Tau**

Legend:
- ◆ Dialysator 3  300 Tau  —Linear  (Tau)
- ■ Dialysator 3  400 Tau  ---Linear  (Tau)
- ▲ Dialysator 3  200 Int  --Linear  (Tau)

Clearance (mit Rezirkulation)

Dialysator-Clearance (ohne Rezirkulation)     Extinction (mit Rezirkulation)

Extinction (ohne Rezirkulation)

Figur 16

Figur 17

BF = 300 ml/min & R = 20%

BF = 240 ml/min & R = 0%

$I_{300}$ & R = 20%

$I_{300}$ & R = 0%

$I_{240}$ & R = 0%

Figur 18

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0886529 B1
- US 7815852 B2
- DE 19702441 C1
- DE 19528907 C1
- DE 102007056475 A1
- WO 0024440 A1
- DE 69916053 T2

**In der Beschreibung aufgeführte Nicht-Patentliteratur**

- **SAN MIGUEL, S ; CHOW, J.** Vascular dialysis access flow measurement: Early intervention through early detection. *J Renal Care,* 2009, 185-191
- **KRIVITSKI, N.** Theory and validation of access flow measurements by dilution technique during hemodialysis. *Kidney International,* 1995, vol. 48, 244-250